# EUROPÄISCHE PATENTANMELDUNG

(11) **EP 3 578 146 A1**
(43) Veröffentlichungstag der Anmeldung: **11.12.2019**
(21) Anmeldenummer: 18176545.4
(22) Anmeldetag: 07.06.2018
(51) Int. Cl.: A61F 7/00

(54) **MOBILE VORRICHTUNG ZUR BEHANDLUNG VON JUCKREIZ MIT SCHNITTSTELLE**

(71) Anmelder: Dermapharm AG, 82031 Grünwald (DE)
(72) Erfinder: BÜNGER, Daniel, 41063 Münchengladbach (DE)
(74) Vertreter: Hertin und Partner Rechts- und Patentanwälte PartG mbB

(57) **Zusammenfassung**

In einem ersten Aspekt betrifft die Erfindung eine Vorrichtung zur hyperthermischen Behandlung von Juckreiz umfassend mindestens eine Behandlungsfläche, welche durch Erhitzen mindestens eines Heizelements auf eine Behandlungstemperatur von 40 °C bis 65 °C aufheizbar ist und durch eine Regelvorrichtung steuerbar ist, wobei die Vorrichtung mindestens ein Interface aufweist und konfiguriert ist für eine Stromversorgung der Vorrichtung durch ein Mobilgerät und/oder zum Datenaustausch mit einem Mobilgerät und/oder zur Steuerung durch ein Mobilgerät über das Interface.

In einem weiteren Aspekt betrifft die Erfindung ein System aus Vorrichtung und Mobilgerät, wobei die Vorrichtung konfiguriert ist für eine Stromversorgung durch das Mobilgerät und/oder zum Datenaustausch mit dem Mobilgerät und/oder zur Steuerung durch das Mobilgerät über ein Interface.

## Beschreibung

In einem ersten Aspekt betrifft die Erfindung eine Vorrichtung zur hyperthermischen Behandlung von Juckreiz umfassend mindestens eine Behandlungsfläche, welche durch Erhitzen mindestens eines Heizelements auf eine Behandlungstemperatur von 40 °C bis 65 °C aufheizbar ist und durch eine Regelvorrichtung steuerbar ist, wobei die Vorrichtung mindestens ein Interface aufweist und konfiguriert ist für eine Stromversorgung der Vorrichtung durch ein Mobilgerät und/oder zum Datenaustausch mit einem Mobilgerät und/oder zur Steuerung durch ein Mobilgerät über das Interface.

In einem weiteren Aspekt betrifft die Erfindung ein System aus Vorrichtung und Mobilgerät, wobei die Vorrichtung konfiguriert ist für eine Stromversorgung durch das Mobilgerät und/oder zum Datenaustausch mit dem Mobilgerät und/oder zur Steuerung durch das Mobilgerät über ein Interface.

### Hintergrund und Stand der Technik

Juckreiz (Pruritus) ist eine subjektiv unangenehme, auf die Haut oder Schleimhaut bezogene Sinneswahrnehmung. Sie kann lokal begrenzt sein oder aber den ganzen Körper betreffen. Häufig geht Juckreiz mit einem brennenden, stechenden oder kribbelnden Gefühl einher, welches die betroffene Person oftmals durch Kratzen, Scheuern, Rubbeln, Drücken, Kneten oder Reiben versucht zu lindern. Daher kommt es bei Juckreiz gehäuft zu weiteren pathologischen Erscheinung der Haut wie Kratzspuren, offene Wunden, Krustenbildung und Hautinfektionen. Die Fachwelt geht davon aus, dass Juckreiz über Schmerzrezeptoren der Haut vermittelt wird und über das vegetative Nervensystem zum Gehirn geleitet wird. Die Ursachen von Juckreiz können sehr vielfältig sein. Neben trockener Haut, fehlender Feuchtigkeitszufuhr oder Allergien kann Juckreiz auch durch äußere Einwirkungen und Hautreizungen, wie z.B. durch Stiche von Mücken oder nach Kontakt mit Nesseltieren entstehen. Juckreiz kann eine Reaktion auf chemische, mechanische oder thermische Reize sein. Es können als Folge der äußerlichen Reizung, wie z.B. durch Einwirkungen chemischer Substanzen, z.B. Histamin (Mückenstich), Apamin (Bienenstich), durch allergische Immunreaktion, durch Druck oder Reibung oder auch durch Wärme oder Sonnenbestrahlung, Quaddeln, Urtikaria und andere mit Juckreiz verbundene Hautreaktionen hervorgerufen werden. Aus medizinischer Sicht spannen die Ursachen oder Grunderkrankungen, welche zu Juckreiz führen ein breites Spektrum dermatologischer und internistischer Erkrankungen auf.

Zur medikamentösen Behandlung von den Symptomen des Juckreizes sind eine Reihe von Medikamenten oder kosmetischen Produkten bekannt. So werden ätherische Öle insbesondere umfassend Menthol, Thymol oder Campher verwandt, um kurzfristig eine Kühlung zu erzeugen. Auch Hautpflegemittel wie beispielsweise Cremen oder Lotion können durch eine Erhöhung des Feuchtigkeitsgehaltes der Haut eine schmerzlindernde Wirkung entfalten. Darüber hinaus stellen Antihistaminika hilfreiche Therapiemöglichkeiten dar, welche beispielsweise die Gabe von Dimetindenmaleat oder Mepyramin umfassen. Weiter Medikamente betreffen topische Glucocorticoide, Anästhetika, Zinksalben, Calcineurinhemmer oder Capsaicin. Zur Behandlung von Wespen oder Bienenstichen werden weiterhin die Einstichstellen auch mit Salmiakgeist behandelt, welches jedoch nur zu einer kurzfristigen Linderung des Juckreizes führt und auch die Schwellung nur im geringen Maße zurückgehen lässt.

Aus dem Stand der Technik ist es jedoch auch bekannt durch Einbringung einer Wärmemenge in den Einstich von Insekten das Auslösen eines Juckreizes zu mindern. Eine Vorrichtung für eine lokale, thermische Behandlung insbesondere von Mückenstichen wird in der EP 1231875 B1 beschrieben. Die Vorrichtung weist eine Heizplatte mit einer Größe von ca. 0,2 cm² auf, welche auf eine Temperatur zwischen 50°C und 65°C gebracht wird, während die Heizplatte den Insektenstich kontaktiert. Durch diese hyperthermische Behandlung lässt sich der Juckreiz nachhaltig lindern. Zum einen resultiert die Wärmeapplikation in einem Abbau der thermolabilen Giftstoffe der Insekten, welche den Juckreiz auslösen. Zum anderen führt die Wärmeübertragung zu einer Überlagerung des Juckreizes durch andere temperaturabhängige Hautempfindungen. Durch derartige Behandlungen können somit weiterhin sekundäre Schädigungen der Haut, beispielsweise eine Entzündung des Insektenstiches durch Kratzen, wirksam vermieden werden. Somit wird durch die hyperthermische Behandlung auch das mit einem Insektenstich einhergehende Auftreten von Quaddeln wirksam reduziert.

Die Anwendungsmöglichkeiten einer hyperthermischen Behandlung erstrecken sich weiterhin auf Herpeserkrankungen. Aus der DE 102005002946 A1 ist eine Vorrichtung zur Behandlung von Herpeserkrankungen bekannt. Die Vorrichtung umfasst eine Heizplatte mit einer bevorzugten Größe von 20 mm², welche für eine Behandlungsdauer von vorzugsweise 10 - 15 sec auf 49 °C - 53 °C erhitzt wird. Während der Behandlungszeit kontaktiert die Heizplatte die betroffene Hautpartie der Lippen, beispielsweise die gerötete Stelle oder aber die Position an der sich bereits Bläschen gebildet haben. Die Hitzeapplikation führt zum einen zu einer Eindämmung der Vermehrung der ursächlichen Krankheitserreger durch eine neutralisierende Wirkung auf die Herpes-simplex-Viren. Zum anderen kommt es durch die kurzzeitige Wärmebehandlung zu einer Überlagerung des Juckreizes der Herpeserkrankung durch die Stimulation temperatursensitiver Nerven. Die Vorrichtung zeichnet sich somit durch eine Reduktion der Symptome der Herpeserkrankung wie z.B. Brennen, dem Auftreten von Schwellungen, einer Rötung oder eines Juckreizes aus.

Aus dem Stand der Technik aus der US 2007/0049998 A1 ist ebenfalls ein Gerät zur hyperthermischen Behandlung von Insektenstichen bekannt, dass eine Behandlungstemperatur von 50°C vorsieht. Dieses Gerät hat den Nachteil, dass bei dieser Temperatur Prozesse, die den Juckreiz lindern, noch nicht oder nicht richtig angestoßen werden. Für die hyperthermische Behandlung wesentliche Prozesse, die zur Linderung der Symptome von Insektenstichen, Herpeserkrankungen, Quallenstichen oder anderen mit Juckreiz einhergehenden Erkrankungen beitragen, werden teilweise erst in einem Temperaturbereich zwischen 50 °C und 56 °C, insbesondere zwischen 50 °C und 53 °C, aktiviert.

Die aus dem Stand der Technik bekannten Vorrichtungen zur hyperthermischen Behandlung zeichnen sich durch vielfältige Einsatzmöglichkeiten zur Linderung der Symptome von Insektenstichen, Herpeserkrankungen, Quallenstichen oder anderen mit Juckreiz einhergehenden Erkrankungen aus. Die Vorrichtungen weisen jedoch auch Nachteile auf.

Insbesondere ist im Stand der Technik kein Gerät bekannt, welches die Vorzüge moderner Mobilgeräte, beispielsweise hohe Rechenleistung, Vernetzung, aber auch die Fähigkeit, als Energieträger für physikalische Anwendung zu dienen, mit einer Vorrichtung zur Behandlung von Pruritus verbindet. Mobilgeräte, insbesondere Smartphones, werden heute nicht nur für Ihre ursprüngliche Bestimmung, beispielsweise zum Telefonieren, verwendet. Es ist vielmehr heutzutage üblich, in den kleinen tragbaren Geräten so viele Anwendungen wie möglich zu vereinen. So werden heutige Smartphones ebenfalls als Fotoapparate, Abspielgeräte, Reader, zum Computerspielen, als Navigationsgerät und für vieles mehr verwendet. Für medizinische Anwendungen, insbesondere bei der Behandlung von Juckreiz, spielen Mobilgeräte bislang keine Rolle. Bisher fehlen hierzu insbesondere in das Mobilgerät integrierte oder mit diesem verbundene Vorrichtungen, welche physikalische Wirkungen verursachen können.

Als weiterer Nachteil kann es bei den bekannten Vorrichtungen in Ausnahmefällen zu einer Überschreitung der gewünschten Behandlungstemperatur kommen. Im Stand der Technik ist es bekannt die Behandlungstemperatur mithilfe von Temperatursensoren zu überwachen. Durch Beschädigungen des Gerätes, beispielsweise durch das Eintreten von Feuchtigkeit, kann es jedoch zu einer Beeinträchtigung des Regelkreislaufes der Überwachungselektronik kommen. Dies ist insbesondere der Fall, wenn die Überwachung der Behandlungstemperatur in den regulären Steuerkreis eingebunden ist. In diesem Fall ist es nicht auszuschließen, dass es zu einer Überhöhung der Temperatur über die gewünschte Behandlungstemperatur hinaus kommt. In Abhängigkeit von der Kontaktposition der Heizplatte bzw. Behandlungsfläche treten hierdurch unerwünschte Nebenwirkungen auf. Selbst bei einer kurzzeitigen Temperaturerhöhung von über 65°C kann es zu nachhaltigen Schädigungen der betreffenden Hautpartien kommen. Dies ist insbesondere der Fall für empfindliche Hautpartien, wie beispielsweise die Lippen während einer Herpesbehandlung oder aber auch dünnere Hautpartien, an welchen Insektenstiche vorliegen.

Aus der US 2007/0049998 A1 ist ein Gerät zur hyperthermischen Behandlung von Hautbeschwerden bekannt, welches eine Behandlungsfläche über ein temperaturgeregeltes Heizelement auf Temperaturen von 38 °C - 67 °C für eine Dauer von mindestens 5 Sekunden, typischerweise jedoch über einen längeren Zeitraum, aufheizt und zur Sicherung gegen Überhitzen eine Schmelzsicherung verwendet. Diese Art der Absicherung gegen Überhitzen hat den Nachteil, dass bei einem Auslösen durch Überhitzen ein Austausch der Sicherung erfolgen muss. Außerdem ist kein redundanter Sicherheitsmechanismus vorhanden und bei Versagen der Schmelzsicherung droht eine Überhitzung der Behandlungsfläche über einen längeren Zeitraum. Des Weiteren werden Temperaturen ab 60 °C oder darüber hinaus, besonders über einen längeren Zeitraum von einigen Sekunden oder länger, als sehr unangenehm empfunden und können zu Hautschädigungen führen. Dies kann mindestens dazu führen, dass der Behandlungserfolg gefährdet wird, weil Behandlungen aufgrund eines unangenehmen Hautgefühls durch die hohen Temperaturen frühzeitig abgebrochen werden und somit der Therapieerfolg gefährdet wird. Dem Gerät liegt der therapeutische Gedanke zu Grunde, durch Wärmeanwendung Keime zu zerstören und Reizerreger der Haut abzutöten. Die herfür benötigten Behandlungsdauern und/oder-temperaturen sind allerdings nicht geeignet, Juckreiz durch gezielte Stimulation bestimmter Rezeptoren und die Modifikation des Immunsystems nachhaltig zu lindern. Temperaturen unterhalb von 42 °C sind wiederum ungeeignet, über eine Wärmeempfindung hinaus Effekte therapeutischer Art zu erzielen.

US 2011/0184502 A1 beschreibt ein Heizkissen für diverse, teils medizinische Anwendungen, welches Temperaturen von 38 °C - 71 °C über mindestens mehrere Minuten elektrisch erzeugt. Es werden nicht rückstellende Thermosicherungen in Serie als redundantes Sicherheitsfeature vorgeschlagen. Somit ist zwar ein redundanter Sicherheitsmechanismus vorhanden, dieser ist jedoch nicht reversibel und muss nach dem Auslösen ausgetauscht werden. Ein zweiter Nachteil bei der Verwendung von thermischen Sicherungen ist, dass diese erst bei Anliegen einer Temperatur oberhalb eines Schwellenwerts abschmelzen. Somit reagieren thermische Sicherungen erst bei Anliegen dieser kritischen Temperatur nach einer gewissen Reaktionszeit und somit gegebenenfalls zu spät im Vergleich zu einer Schmelzsicherung. Eine Schmelzsicherung löst bereits bei einem elektrischen Strom oberhalb eines Schwellenwertes aus, der eine zu hohe Temperatur verursachen könnte, wenn er zu lange fließt. Darüber hinaus ist sowohl der Temperaturbereich als auch die Dauer des Heizprozesses für eine Vielzahl von Anwendungen sicherlich relevant, jedoch ungeeignet, Pruritus durch Hitzeanwendung nachhaltig zu lindern.

Es wäre somit wünschenswert eine Vorrichtung bereitstellen zu können, welche die Vorzüge der hyperthermischen Behandlung für die Vielzahl der genannten Erkrankungen umsetzt und gleichzeitig Sicherheitsrisiken minimiert, bevorzugt indem ein redundanter, aber auch praktischer und hohen Standards genügender Sicherheitsmechanismus gegen Überhitzen verwendet wird.

### Aufgabe der Erfindung

Eine Aufgabe der Erfindung war es, die Nachteile des Standes der Technik zu beseitigen. Insbesondere war es Aufgabe der Erfindung, die Vorzüge von Mobilgeräten, welche Vernetzung, Rechenleistung und die Bereitstellung elektrischer Energie bieten, mit den Vorteilen einer Vorrichtung zu Behandlung von Juckreiz zu verbinden.

### Kennzeichnung der Erfindung

In einem ersten Aspekt betrifft die Erfindung eine Vorrichtung zur hyperthermischen Behandlung von Juckreiz, umfassend mindestens eine Behandlungsfläche, welche durch Erhitzen mindestens eines Heizelements auf eine Behandlungstemperatur von 40 °C bis 65 °C aufheizbar ist und durch eine Regelvorrichtung steuerbar ist, dadurch gekennzeichnet, dass die Vorrichtung mindestens ein Interface aufweist und konfiguriert ist für eine Stromversorgung der Vorrichtung durch ein Mobilgerät und/oder zum Datenaustausch mit einem Mobilgerät und/oder zur Steuerung durch ein Mobilgerät über das Interface.

In einem weiteren Aspekt betrifft die Erfindung ein System aus Vorrichtung und Mobilgerät, wobei die Vorrichtung konfiguriert ist für eine Stromversorgung durch das Mobilgerät und/oder zum Datenaustausch mit dem Mobilgerät und/oder zur Steuerung durch das Mobilgerät über ein Interface.

### Definitionen und bevorzugte Ausführungsformen

In einem ersten Aspekt betrifft die Erfindung eine Vorrichtung zur hyperthermischen Behandlung von Juckreiz umfassend mindestens eine Behandlungsfläche, welche durch Erhitzen mindestens eines Heizelements auf eine Behandlungstemperatur von 40 °C bis 65 °C aufheizbar ist und durch eine Regelvorrichtung steuerbar ist, dadurch gekennzeichnet, dass die Vorrichtung mindestens ein Interface aufweist und konfiguriert ist für eine Stromversorgung der Vorrichtung durch ein Mobilgerät und/oder zum Datenaustausch mit einem Mobilgerät und/oder zur Steuerung durch ein Mobilgerät über das Interface.

Untersuchungen haben erfindungsgemäß ergeben, dass das Einbringen von Wärme durch eine derartige Einrichtung überraschenderweise erfolgreich zur Behandlung von Juckreiz verwendet werden kann.

Dafür wird die erfindungsgemäße Vorrichtung bevorzugt auf die betroffenen Hautpartien aufgelegt. Nach der Kontaktierung der Hautpartie mit der Behandlungsfläche sorgt eine Regelvorrichtung dafür, dass eine erfindungsgemäße Regulation der Temperatur der Behandlungsfläche erfolgt. Die Behandlungstemperatur gibt dabei bevorzugt stets jene Temperatur an, welche an der Hautpartie des Patienten vorliegt. Bevorzugt wird die Behandlungsfläche hierzu zunächst in einer Aufheizphase auf eine Behandlungstemperatur zwischen 40 °C und 65 °C geführt. Es ist bevorzugt, dass die Aufheizphase keinem längeren Zeitraum bedarf. Bevorzugt sollte die Aufheizphase nicht mehr als 10 sec, besonders bevorzugt nicht mehr als 3 sec betragen. Im Anschluss an die Aufheizphase wird die Temperatur der Behandlungsfläche bevorzugt bei der vorbestimmten Behandlungstemperatur gehalten. Die Behandlungstemperatur entspricht bevorzugt einer konstanten Temperatur, welche im genannten Bereich zwischen 40 °C und 65 °C liegt.

Diese Behandlungstemperatur wird während der Behandlungsphase bevorzugt konstant gehalten. Es kann aber auch bevorzugt sein, dass die Behandlungstemperatur nicht konstant gehalten wird. Beispielsweise kann die Behandlungsfläche auch in einer Temperaturrampe auf eine Maximaltemperatur im Bereich der Behandlungstemperatur zwischen 40 °C und 65 °C geführt werden. Im Anschluss daran kann es bevorzugt sein, dass die Temperatur kurzzeitig unterhalb des Bereiches der Behandlungstemperatur gesenkt wird. Anschließend kann die Temperatur wieder steigen, so dass ein zeitlicher Temperaturverlauf in Form einer Rampe entsteht. Diese Vorzugsvariante hat sich bei einigen zu Juckreiz führenden Hauterkrankungen überraschenderweise als vorteilhaft gegenüber dem Beibehalten einer konstanten Behandlungstemperatur erwiesen. So kann es beispielsweise von Vorteil sein, die Maximaltemperatur nur für sehr kurze Zeit über eine ansteigende Temperatur zu erreichen und danach wieder auf eine Rampe etwas abzukühlen.

Die Behandlungsphase bezeichnet bevorzugt den Zeitraum, bei welchem die Temperatur im Bereich der Behandlungstemperatur von 40 °C bis 65 °C ist. Bevorzugt dauert die Behandlungsphase 2 bis 20 sec, besonders bevorzugt dauert die Behandlungsphase zwischen 2 sec und 12 sec, ganz besonders bevorzugt zwischen 3 sec und 6 sec. Es ist besonders bevorzugt, dass die Behandlungsphase einen zusammenhängenden Zeitraum bezeichnet. Es kann aber auch sein, dass die Behandlungsphase durch das Führen der Temperatur, welches zeitlich die Form einer Rampe aufweist, kurzzeitig unterbrochen wird. In diesem Falle wird als Zeitraum der Behandlungsphase bevorzugt diejenige Zeit verstanden, während welcher die Temperatur der Behandlungsfläche im Bereich der Behandlungstemperatur von 40 °C bis 65 °C liegt.

Es kann bevorzugt sein, dass die die Behandlungsphase nicht im Voraus definiert ist, sondern individuell angepasst wird, beispielsweise durch die Regelvorrichtung, z. B. in Verbindung mit anderen extern zur Verfügung gestellten Parametern. Es kann ebenso bevorzugt sein, dass die Behandlungsphase solange andauert, wie ein Benutzer einen Regelknopf zur Aufrechterhaltung der Behandlung betätigt. Die Behandlungsphase kann im Folgenden synonym mit der Behandlungsdauer verwendet werden.

Im Sinne der Erfindung bezeichnet die Behandlungsfläche bevorzugt diejenige Fläche der Vorrichtung, welche während der Behandlung auf die Behandlungstemperatur aufgeheizt wird und in direktem thermischen Kontakt mit der Hautpartie steht. Die Behandlungsfläche kann eine zusammenhängende Fläche darstellen. Es kann auch bevorzugt sein, dass die Behandlungsfläche aus mehreren nicht zusammenhängenden Teilflächen besteht. Die Größe der Behandlungsfläche hängt bevorzugt von der Erkrankung und der Größe der von den Symptomen der Juckreizerkrankung betroffenen Hautpartien ab. Bei Insektenstichen ist die Größe der Behandlungsfläche zwischen 10 mm² und 100 mm², besonders bevorzugt zwischen 20 mm² und 60 mm². Bei der Behandlung von Herpes beträgt die Behandlungsfläche bevorzugt zwischen 10 mm² und 80 mm², besonders bevorzugt zwischen 20 mm² und 50 mm². Weiterhin ist es besonders bevorzugt, dass die Behandlungsfläche für diese kleineren Hautpartien kreisförmig ist. Durch die so gewählten Größen und Geometrien der Behandlungsfläche kann eine der Ursache optimal angepasste Behandlung erfolgen, welche Effizienz und Wohlbefinden optimiert und so zu einem nachhaltigeren Behandlungserfolg beiträgt.

Die Größe der Behandlungsfläche bezieht sich bevorzugt jeweils auf die gesamte Kontaktfläche, über welche eine Hautpartie einen Wärmeimpuls erfährt. Im Falle einer Behandlungsfläche, welche aus mehreren Teilflächen besteht, entspricht die Größe der Behandlungsfläche bevorzugt der Summe der einzelnen Teilflächen. Eine solche Aufteilung in Teilflächen kann bei bestimmten Erscheinungsformen von Herpes vorteilhaft sein ebenso wie bei der Behandlung bestimmter Körperstellen.

Es ist bevorzugt, dass die Behandlungsfläche mit Hilfe von mindestens einem Heizelement auf die gewünschte Behandlungstemperatur gebracht wird. In einer bevorzugten Ausführungsform entspricht die Behandlungsfläche der Oberfläche einer Heizplatte, welche mit Hilfe eines Heizelementes erhitzt wird, wobei zum Beispiel ein Halbleiter-Bauelement zum Einsatz kommen kann. Die Behandlungsfläche kann auch eine homogene Materialfläche bezeichnen, welche durch mehrere Heizelemente temperiert wird. Beispielsweise kann es bevorzugt sein, zwei oder vier Heizelemente zu verwenden, um die Behandlungsfläche besonders homogen und schnell auf die Behandlungstemperatur zu führen.

Es ist bevorzugt, dass eine Regelvorrichtung das Erhitzen des Heizelementes derart regulieren kann, dass die Behandlungstemperatur an der Behandlungsfläche vorliegt. So kann eine optimale Kontrolle der Behandlungstemperatur gewährleistet werden und ein ungewünschtes Überhitzen der Behandlungsfläche unterbunden werden.

Im Sinne der Erfindung ist die Regelvorrichtung bevorzugt ein integrierter Schaltkreis, Prozessor, ein Prozessorchip, Mikroprozessor oder ein Mikrokontroller, welcher konfiguriert ist, um die Temperatur der Behandlungsfläche mit Hilfe des mindestens einen Heizelements gemäß der vorgegebenen Werte für die Behandlungstemperatur zu regulieren. Eine solche Regelvorrichtung zeichnet sich durch Kompaktheit, Zuverlässigkeit, Kosteneffizienz, geringen Stromverbrauch und hohe Regeleffizienz aus. Diese Regelvorrichtung kann beispielsweise von der Vorrichtung umfasst sein. Es kann ebenso bevorzugt sein, dass die Regelvorrichtung Teil des Mobilgeräts ist, insbesondere, wenn eine Steuerung der Vorrichtung durch das Mobilgerät über die Schnittstelle angestrebt wird.

Das mindestens eine Heizelement ist ein Bauelement, für welche verschiedene Ausführungsformen aus dem Stand der Technik hinreichend bekannt sind. So kann das Heizelement einen Leistungswiderstand umfassen, bei welchem in Abhängigkeit des Stromflusses eine wohldefinierte Temperatur generiert wird. Bevorzugt kann zur quantitativen Steuerung des Stromflusses durch das Heizelement ein Feldeffekttransistor (FET) verwandt werden. Es kann aber auch bevorzugt sein, einen FET selbst als Heizelement einzusetzen. Hierbei wird die Energiedissipation im Transistor genutzt, um Wärme zu generieren und die Behandlungsfläche auf die Behandlungstemperatur zu bringen. FETs sind als Heizelemente besonders bevorzugt, da diese aufgrund ihrer geringen Größe eine kleine Dimensionierung erlauben. Weiterhin sind FETs besonders reaktiv und gewährleisten durch eine sehr dynamische Wärmeerzeugung und Wärmeabgabe ein besonders schnelles Ansprechverhalten der Heizelemente.

Die beschriebene Einrichtung umfasst bevorzugt ein Heizelement, das beispielsweise als elektrische Heizplatte in Form einer runden Scheibe mit einem Durchmesser von etwa 5 mm ausgeführt ist. Das Heizelement wird bevorzugt durch eine Spannungsquelle gespeist. Mit dem Heizelement verbunden ist bevorzugt ein Temperatursensor. Das vom Temperatursensor erzeugte elektrische Signal wird vorteilhafterweise der Regelvorrichtung zugeführt, mit der die Temperatur und die Dauer der Aufrechterhaltung der maximalen Temperatur der Behandlungsfläche gesteuert wird. Beispielsweise durch das Betätigen eines Tasters wird mittels der Steuereinrichtung im Heizelement die Aufheizphase gestartet. Sobald das vom Sensor abgegebene elektrische Signal die der vorgegebenen maximalen Temperatur an dem Heizelement die entsprechende Größe erreicht hat, beginnt bevorzugt die Behandlungsphase. Dazu wird in der Steuereinrichtung, die bevorzugt eine Zeit- und Temperaturregelung enthält, die Zeitsteuerung ausgelöst. Während der vorgegebenen Dauer der Behandlungsphase gewährleistet die Temperaturregelung in erster Linie die Aufrechterhaltung der maximalen Temperatur und verhindert gleichzeitig deren Überschreiten. Nach dem Ablauf der Behandlungsphase wird der Heizvorgang vorzugsweise beendet und Temperatur der Heizplatte passt sich der Umgebungstemperatur an. Die Behandlungsfläche wird bei dieser Einrichtung in der Aufheizphase bevorzugt auf eine maximale Temperatur aus einem Bereich von 40 bis 65 °C erhitzt, wobei eine Toleranz von ± 3 °C eingehalten wird. Dabei kann die maximale Temperatur in einer Heizphase für einen Zeitraum von bevorzugt 2 bis 20 sec, besonders bevorzugt 3 bis 20 sec, insbesondere 10 bis 15 sec, aufrechterhalten werden. Es kann ebenfalls bevorzugt sein, die Behandlungsphase individuell anzupassen.

Bevorzugt kann die Regelvorrichtung durch die Vorgabe der Stromzufuhr zum Heizelement kontrollieren, welche Temperatur an der Behandlungsfläche vorliegt. Beispielsweise kann mit Hilfe einer Kalibration die Korrelation zwischen Stromfluss und/oder Spannung an dem Heizelement mit der Temperatur an der Behandlungsfläche ermittelt werden, sodass auf Basis der Kalibrierung stets eine gewünschte Behandlungstemperatur zwischen 40 °C und 65 °C eingestellt werden kann. Es kann aber auch bevorzugt sein, die Behandlungstemperatur durch die Regelvorrichtung mit Hilfe einer Feedbackschleife zu regulieren. So kann es bevorzugt sein, einen Temperatursensor einzusetzen, welcher die Temperatur der Behandlungsfläche misst, wobei die Regelvorrichtung anhand der Temperaturdaten die Stromzufuhr zum Heizelement reguliert. Zu diesem Zweck kann die Regelvorrichtung beispielsweise einen Mikroprozessor umfassen, welcher Messdaten auswerten und Stromparameter festlegen kann. So kann die Temperatur sehr effizient und zuverlässig gesteuert werden.

Durch die Regulation der Behandlungsfläche auf einer Temperatur zwischen 40°C und 65 °C während einer Behandlungsphase wird Wärme auf wohldefinierte Art übertragen. Hierdurch können Schmerzen und weitere unangenehme Empfindungen wie bspw. ein Ziehen oder Juckreiz überraschend wirksam gelindert werden. Der Effekt kann auch bei Insektenstichen zum Tragen kommen, bei denen die Linderung zusätzlich aus einer thermischen Neutralisation der Gifte der Insekten beruht. Zum anderen bewirkt die Wärme eine Nervenstimulation, welche die subjektive Wahrnehmung von Schmerzen oder Juckreiz an den betreffenden Stellen stark reduziert. Die Wärmeübertragung führt überraschend zu einer Überlagerung der unangenehmen Empfindung durch andere temperaturabhängige Hautempfindungen. Entgegen konventionellen Methoden zur Behandlung von Herpes wird dabei zusätzlich eine Regulation der Schmerzrezeptoren anvisiert und durch die bevorzugte Wärmebehandlung die freien Nervenenden der C-Fasern aktiviert. Die C-Fasern bezeichnen insbesondere die langsam leitenden Nervenfasern des somatosensiblen Systems und sind für die Schmerzwahrnehmung verantwortlich. Hierbei kommt insbesondere den freien Enden der C-Fasern, welche auch als Nozizrezeptoren bezeichnet werden, eine wichtige Rolle zu. Die Nervenenden der Fasern werden durch Gewebshormone (z. B. Histamin, Serotonin, Substanz P) aktiviert. Auch könnten Mastzellen in der Nähe der Nervenenden durch Ausschüttung des Mediators Tryptase an dem Prozess beteiligt sein. Insbesondere wird die Erkenntnis über den Wirkmechanismus bei Herpes ausgenutzt, um durch eine Wärmebehandlung in überraschender Weise die Sinneswahrnehmung, welche durch die Fasern ausgelöst werden, zu regulieren. Neben dem besonders bevorzugten Einsatz der Vorrichtung zur Behandlung von Juckreizerkrankungen z.B. nach Kontakt mit giftigen Nesseltieren oder Pflanzen, wie Brennnesseln oder bei Insektenstichen und -bissen erlauben die genannten Temperaturparameter und bevorzugt auch Zeiträume auf überraschende Weise auch die Behandlung von Herpes. Es können darüber hinaus thermolabile Gifte von Insektenstichen neutralisiert werden.

Es wurde darüber hinaus erkannt, dass eine besonders starke Überlagerung der unangenehmen Empfindungen erreicht werden kann, wenn in den betreffenden Hautpartien lokal die Thermo- und Capsaicinrezeptoren TRPV1 und TRPV2 zeitgleich aktiviert werden. Der TRPV1 ist bei akutem Hitze-induzierten Schmerz in gesunder Haut beteiligt und reguliert beispielsweise das Heißempfinden bei Temperaturen um 45° bis 50 °C. Bei besonders starken schmerzhaften Hitzereizen, welche ab Temperaturen von über 52 °C eintreten wird darüber hinaus der TRPV2 aktiviert. Die Aktivierungsschwelle des TRPV1 liegt zwischen 40°C und 45°C, wohingegen die des TRPV2 zwischen 50°C und 53° beträgt (Yao et al 2011, Somogyi et al. 2015, Cohen et al. 2014, Mergler et al. 2014).

Es ist bevorzugt, dass die Vorrichtung mindestens ein Interface aufweist und konfiguriert ist für eine Stromversorgung der Vorrichtung durch ein Mobilgerät und/oder zur Datenübertragung mit einem Mobilgerät und/oder zur Steuerung durch ein Mobilgerät über das Interface.

Ein Interface oder, synonym, eine Schnittstelle, bedeutet, dass die Vorrichtung dafür konfiguriert ist, mit einer anderen Vorrichtung zu interagieren und/oder verbunden zu werden. Es kann sich um einen physischen Ort handeln, an dem eine Verbindung der Vorrichtung mit einem anderen Gerät möglich ist. Es kann sich aber ebenso um eine andere, optionale Verbindungsmöglichkeit der Vorrichtung handeln, mit einer anderen Vorrichtung Signale und/oder Energie auszutauschen. Dabei kann es sich bevorzugt um elektrische Signale handeln oder um Signale, welche von der Vorrichtung in elektrische Signale umgewandelt werden können. Es kann sich ebenso bevorzugt um Energie handeln, welche ausgetauscht wird bzw. übertragen wird. Besonders bevorzugt kann es sich dabei um elektrische Energie handeln oder um Energie, welche bevorzugt von der Vorrichtung in elektrische Energie umgewandelt werden kann.

Bei der anderen Vorrichtung handelt es bevorzugt um ein Mobilgerät. Ein Mobilgerät kann beispielsweise ein Laptop, ein Mobiltelefon, ein Smartphone, ein Tablet-Computer, ein Notebook, eine Smartwatch und/oder eine Powerbank sein.

Eine Verbindung, welche über eine solche Schnittstelle ermöglicht wird, umfasst bevorzugt drei physische Elemente: eine Schnittstelle des Mobilgeräts, die Schnittstelle der Vorrichtung sowie einen Übertragungskanal zwischen diesen beiden. Der Übertragungskanal kann beispielsweise eine kabelbasierte Verbindung sein und/oder ein drahtloser Übertragungskanal, der auf der Übersendung von Energie beispielsweise durch elektromagnetische Strahlung beruht.
Es kann auch bevorzugt sein, dass die zwei Schnittstellen in Form von Buchse und dazu passendem Stecker ausgeführt sind und so eine direkte Verbindung herstellbar ist, welche bevorzugt des Weiteren eine mechanisch stabile Verbindung zwischen Vorrichtung und Mobilgerät ermöglicht. In diesem Fall und bei einer kabelbasierten Verbindungsmöglichkeit würde die Schnittstellte bevorzugt eine Buchse und/oder einen Stecker umfassen.

Je nachdem, ob die Schnittstelle drahtlos oder kabelgebunden ist oder für eine direkte Steckverbindung mit Stecker und Buchse vorgesehen ist, umfasst die Schnittstelle, unterschiedlich gestaltete Elemente.

Bei der Schnittstelle kann es sich um eine standardisierte Schnittstelle handeln, wie beispielsweise Bluetooth, Lightning, USB, WLAN etc. handeln. Es kann sich jedoch auch um eine individuell für die Vorrichtung entwickelte Schnittstelle handeln. Die Schnittstelle kann drahtlos oder kabelgebunden sein. Ein Kabel kann sowohl zur Übertragung elektrischer Ladungsträger geeignet sein, beispielsweise ein Kupferkabel sein, es kann aber ebenso ein Kabel beispielsweise zur Übertragung optischer Signale, zum Beispiel ein Glasfaserkabel verwendet werden. Eine drahtlose Übertragung kann bevorzugt auf der Übertragung elektromagnetischer Wellen im kompletten Spektralbereich basieren, es können beispielsweise Lichtsignale zur drahtlose Übertragung verwendet werden, aber ebenso Kurzwellen, Ultrakurzwellen und/oder Dezimeterwellen.

Handelt es sich bei der Schnittstelle z. B. um USB, ist mit Schnittstelle der Vorrichtung bevorzugt eine zur Verbindung geeignete USB-Buchse der Vorrichtung gemeint. Des Weiteren ist bevorzugt gemeint, dass von Seiten der Vorrichtung geeignete Datenwandler, Leitungstreiber, Stromversorgungen und/oder Prozessoren vorhanden sind, um die USB-Buchse bestimmungsgemäß zu nutzen, insbesondere zur Einrichtung einer USB-Verbindung mit einem Mobilgerät. Außerdem soll insbesondere eine Verbindung von elektrischen Komponenten der Vorrichtung mit der Schnittstelle gewährleistet sein. Dieses Beispiel dient der Illustration einer Schnittstelle der Vorrichtung und ist bevorzugt vom Prinzip her auf andere Arten von Schnittstellen der Vorrichtung übertragbar.

Ein Interface kann bevorzugt zur Übertragung von Daten verwendet werden. Dabei ist die Vorrichtung bevorzugt konfiguriert für eine Datenübertragung mit einem Mobilgerät über das Interface. Daten sind bevorzugt Informationen, die von einem elektronischen Datenverarbeitungsgerät ausgegeben, empfangen und/oder verarbeitet werden können. Ein Datenverarbeitungsgerät ist bevorzugt eine Vorrichtung ausgesucht aus der Gruppe Computer, Mikrocomputer, Prozessor, Mikroprozessor, integrierter Schaltkreis, Smartphone, sonstige Mobilgeräte und/oder Regelvorrichtung. Bevorzugt sind Daten in digitalem Format, insbesondere in Bits. Auch analoge Daten sind jedoch vorstellbar. Ebenso können Daten bevorzugt in einem Speicher oder auf einem Speichermedium gespeichert werden.

Eine Datenübertragung mit einem Mobilgerät bezeichnet bevorzugt den Austausch von Daten mit dem Mobilgerät. Dabei können Daten bevorzugt von der Vorrichtung empfangen und/oder gesendet werden. Ebenso können Daten bevorzugt von der Vorrichtung gespeichert und/oder verarbeitet werden.

Um für eine Datenübertragung über die Schnittstelle konfiguriert zu sein, sollte bevorzugt über die Schnittstelle eine Datenleitung, bevorzugt mehrere parallele Datenleitungen zwischen Vorrichtung und Mobilgerät über die Schnittstelle eingerichtet sein. Das bedeutet, dass über Datenleitungen eine Übertragung zwischen Mobilgerät und Schnittstelle sowie zwischen Schnittstelle und weiteren elektrischen Komponenten der Vorrichtung möglich ist. Die Übertragung zwischen Mobilgerät und Schnittstelle kann bevorzugt über eine Schnittstelle des Mobilgeräts erfolgen, welche je nach Schnittstelle beispielsweise kabelgebunden und/oder drahtlos erfolgen kann, wie bereits obenstehend geschildert wurde. Die Übertragung zwischen Schnittstelle der Vorrichtung und den weiteren Komponenten kann beispielsweise durch physische Signalleitungen in Form von Kabeln realisiert werden, welche die Übertragung elektrischer Signale ermöglichen.

Dabei kann bevorzugt sein, dass mindestens eines der Elemente aus Mobilgerät, Schnittstelle des Mobilgeräts, Schnittstelle der Vorrichtung und elektrischen Komponenten der Vorrichtung ein unterschiedliches Datenformat verwendet als mindestens eines der übrigen Elemente aus dieser Gruppe. Daher kann es bevorzugt sein, dass an den Verbindungsstellen dieser Elemente, welche unterschiedliche Formate verwenden, mindestens ein Datenwandler vorliegt, der die Daten in Übertragungsrichtung in ein für das folgende Element geeignetes Datenformat überträgt. Sofern solch ein Datenwandler der Schnittstelle der Vorrichtung zugeordnet ist und/oder in Richtung der Komponenten der Vorrichtung dieser folgt, ist dieser mindestens eine Datenwandler bevorzugt Teil der Vorrichtung.

Zum Senden von Daten umfasst die Vorrichtung und/oder deren Schnittstelle bevorzugt mindestens eine Datensendeeinrichtung. Diese kann Daten der Vorrichtung über die Schnittstelle in Richtung Mobilgerät übertragen. Die Daten können dabei bevorzugt von einer in die Vorrichtung integrierten Regelvorrichtung generiert werden und/oder auf einem Datenspeicher der Vorrichtung vorliegen. Bevorzugt kann die Datensendeeinrichtung auch als Datenwandler agieren bzw. einen solchen umfassen. Einem Fachmann sind geeignete Einrichtungen bekannt und/oder er kann sie routinemäßig einrichten.

Zum Empfangen von Daten umfasst die Vorrichtung bevorzugt mindestens eine Datenempfangseinheit. Diese kann vom Mobilgerät kommende Daten über die Schnittstelle empfangen, diese gegebenenfalls in ein geeignetes Datenformat umwandeln und beispielsweise an eine Regelvorrichtung zur Weiterverarbeitung und/oder ein Speichermedium zum Speichern in geeigneter Weise weitergeben. Ein Fachmann kennt Möglichkeiten, geeignete Einheiten routinemäßig zu realisieren.

Es kann auch bevorzugt sein, dass Datensende- und empfangseinheit in einer gemeinsamen Einheit kombiniert sind.

Wie bereits vorstehend beschrieben, umfasst die Vorrichtung bevorzugt einen Speicher, welcher bei der Datenübertragung genutzt werden kann. Dabei kann es sich beispielsweise um einen Speicher ausgesucht aus der Gruppe umfassend Festkörperspeicher, RAM-Speicher, ROM-Speicher, EPROM-Speicher, EEPROM-Speicher, Flash-Speicher und/oder um andere Speichertechnologien handeln.

Die Daten können dabei parallel und/oder seriell übertragen werden. Bei einer parallelen Übertragung können mehrere digitale Informationseinheiten, sogenannte Bits, gleichzeitig übertragen werden.

Die Daten enthalten bevorzugt die Behandlung betreffende Parameter, welche der Regelvorrichtung übermittelt werden. Bei diesen Parametern kann es sich beispielsweise um Behandlungsparameter wie die Behandlungstemperatur, die Behandlungsdauer und/oder eine zeitliche Abfolge von anzuwendenden Behandlungstemperaturen handeln, um ein bestimmtes zeitliches Temperaturprofil während der Behandlung zu ermöglichen. Diesbezüglich können auch beispielsweise Metadaten an die Regelvorrichtung übermittelt werden, aus welchen von dieser konkrete Behandlungsparameter ermittelt werden. Bei diesen Metadaten kann sich beispielsweise um den Nutzer betreffende Daten handeln. Beispielsweise können Geschlecht, Alter und/oder individuelle Behandlungsvorlieben von Nutzern übertragen werden. Die Übertragung der genannten Daten kann ebenso gut von der Vorrichtung zum Mobilgerät erfolgen, um beispielsweise die vom Benutzer an der Vorrichtung eingestellte Behandlungsdauern und Behandlungstemperaturen an das Mobilgerät zu übertragen und zu diesen Nutzer betreffenden Nutzerdaten zu verknüpfen. Es können durch eine Datenübertragung zwischen der Vorrichtung und einem Mobilgerät weitere relevante Metadaten generiert werden, die beispielsweise Behandlungserfolg, Heilungsverlauf, Häufigkeit der Anwendung usw. zum Inhalt haben. Zusätzlich kann das Mobilgerät, welches beispielsweise ein Smartphone ist, weitere Daten generieren, wie beispielsweise GPS-Positionsdaten, Zeitstempel, Fotos von von Juckreiz betroffenen Hautpartien etc. Diese können alle miteinander verknüpft und/oder korreliert werden, wodurch statistisch relevante Daten erzeugt werden können, die zum Beispiel erfolgreiche Behandlungstherapien betreffen oder aber einen geographisch und/oder zeitlich begrenztes Auftreten einer Insektenplage. Auch sind durch die Verknüpfung von Mobilgerät und Vorrichtung weitere interessante Anwendungen denkbar, wie beispielsweise eine fernmündliche medizinische Behandlung und/oder Beratung.

Das Interface kann bevorzugt ebenso oder auch ausschließlich zur Übertragung von elektrischer Energie verwendet werden. Dabei ist die Vorrichtung bevorzugt konfiguriert für eine Stromversorgung durch ein Mobilgerät über das Interface.

Dafür ist bevorzugt, eine Energieübertragung vom Mobilgerät zur Vorrichtung über das Interface zu ermöglichen. Die Energieübertragung sieht insbesondere die Übertragung elektrischer Energie vor. Die Energie kann aber auch in anderer Form übertragen und dann von der Vorrichtung in elektrische Energie umgewandelt werden. Die Übertragung, insbesondere von elektrischer Energie, kann kabelgebunden stattfinden. In diesem Fall wird das Interface insbesondere gebildet durch eine Möglichkeit der Kabelverbindung zwischen Mobilgerät bzw. dessen Schnittstelle und Vorrichtung. Die Übertragung kann aber auch drahtlos stattfinden, wobei von Seiten der Vorrichtung das hierfür geeignete Interface bevorzugt aus einem Energiewandler besteht, der die drahtlos übertragene Energie in elektrische Energie umwandelt, welcher zur Stromversorgung der Vorrichtung verwendet werden kann. Die am Interface empfangene, übertragene elektrische Energie wird bevorzugt an die Elemente der Vorrichtung übertragen, welche elektrische Energie verbrauchen, wie beispielsweise das Heizelement oder einer eventuell integrierten Regelvorrichtung. Diese Übertragung kann durch geeignete Verbindungen, z. B. durch Kabel, realisiert werden. Hierbei kann ein Energiespeicher, wie er untenstehend beschrieben wird, zwischen- und/oder nachgeschaltet sein. Die Verwendung geeigneter Spannungsregler und/oder Laderegler zum Versorgen und/oder Laden des Energiespeichers und die elektrische Verschaltung einzelner Elemente der Vorrichtung mit der Schnittstelle ist ebenso bevorzugt und von einem Fachmann routinemäßig vornehmbar.

Insbesondere können beide Übertragungen, die von Energie und Daten parallel stattfinden. Es kann auch bevorzugt sein, dass Interface nur zu einer Art von Übertragung, zum Beispiel zur Übertragung von elektrischer Energie zur verwenden. Einige Schnittstellen, wie zum Beispiel USB, sehen eine parallele Spannungs- und Datenübertragung vor. Auch bei einem drahtlosen Interface kann elektrische Energie und/oder Daten, bevorzugt parallel, übertragen werden, beispielsweise durch Induktionsspulen oder durch elektromagnetische Strahlung, welche durch Solarzellen und/oder Photodioden in elektrische Spannung umgewandelt wird.

Die Vorrichtung ist bevorzugt konfiguriert für eine Steuerung durch ein Mobilgerät über das Interface.

Unter Steuerung ist bevorzugt zu verstehen, das Aufheizen des Heizelements unter Beachtung der Behandlungsparameter, wie zum Beispiel Behandlungstemperatur und Behandlungsdauer, zu kontrollieren. Dabei sollen vor allem Messdaten des Temperatursensors und/oder Zeitinformationen verwendet werden. Es kann dabei bevorzugt sein, dass die Vorrichtung selbst keine Regelvorrichtung aufweist und deren Aufgabe durch eine geeignete Hardware und/oder Software des Mobilgeräts übernommen wird. Somit ist in diesem Fall bevorzugt, dass das Mobilgerät eine Regelvorrichtung umfasst. Dabei kann beispielsweise bevorzugt sein, dass die Steuerungssignale, beispielsweise zum Heizen des Heizelements, direkt vom Mobilgerät über das Interface an dieses übertragen werden. Die Steuerung kann beispielsweise durch eine Kontrolle der übertragenen Stromstärke und/oder durch ein pulsweitenmoduliertes Signal seitens des Mobilgeräts vorgenommen werden. Ebenso können bevorzugt die Messdaten des Temperatursensors über die Schnittstelle direkt an das Mobilgerät übertragen und durch dieses direkt oder nach einer geeigneten Signalumwandlung ausgelesen und zur Regelung verwendet werden.

Ebenso kann bevorzugt sein, dass die Vorrichtung einen rudimentären Prozessor, beispielsweise einen Mikroprozessor und/oder einen Datenwandler umfasst, welcher die Steuerbefehle des Mobilgeräts in geeignete Signale zur Steuerung des Heizelements umwandelt. Ebenso können Signale des Temperatursensors in für das Mobilgerät geeignete Signale umgewandelt und an dieses über die Schnittstelle übermittelt werden.

Zur Übermittlung der Steuerdaten über die Schnittstelle kann bevorzugt die vorstehend geschilderte Datenübertragung verwendet werden.

Zur Steuerung durch ein Mobilgerät über das Interface weist die Vorrichtung bevorzugt mindestens eine elektrische Verbindung zwischen Interface und Prozessor und/oder Heizelement und/oder Temperatursensor auf. Diese kann in Form elektrischer Kabel vorgenommen werden. Je nach Realisierung sind zwischen Schnittstelle und den genannten verbundenen Elementen Datenwandler vorgesehen.

So kann die Vorrichtung besonders einfach, kostengünstig und kompakt gehalten werden. Es können vorhandene Steuerungsressourcen des Mobilgeräts, beispielsweise eines Smartphones, genutzt werden. Es kann aber auch von Vorteil sein, dass die Vorrichtung eine Regelvorrichtung umfasst, und nur Behandlungsparameter vom Mobilgerät an die Regelvorrichtung übertragen werden. Eine solche Übertragung kann wahlweise vor jeder Behandlung vorgenommen werden, es kann aber auch bevorzugt sein, dass eine Übertragung nur stattfindet, wenn neue Behandlungsparameter verwendet werden sollen.

Es ist bevorzugt, dass die Vorrichtung die Vorrichtung die Regelvorrichtung umfasst. Die Regelvorrichtung kann dabei vorstehend genannte Eigenschaften aufweisen und in geschilderter Weise zur Steuerung des Heizelements wirken.

In einer bevorzugten Ausführungsform ist die Vorrichtung konfiguriert für eine Parametrierung der Regelvorrichtung durch das Mobilgerät. In dieser Ausführungsform werden Benutzungsparameter vom Mobilgerät über die Schnittstelle auf die Regelvorrichtung der Vorrichtung übertragen. Diese können insbesondere Behandlungstemperatur und -dauer umfassen. So können individuell vor jeder Behandlung für den jeweiligen Benutzer und sein Leiden geeignete Parameter übertragen werden. Es kann eine besonders individuelle Behandlung erfolgen und der Behandlungserfolg dadurch erhöht werden. Es können auch über das Mobilgerät bevorzugte Parameter einzelner Benutzer ausgetauscht werden. Hat beispielsweise ein Benutzer bei Mückenstichen besonders gute Erfahrungen mit einer bestimmten Behandlungstemperatur und Behandlungsdauer gemacht, kann er seine diesbezüglichen Parameter anderen Benutzern zur Verfügung stellen. Diese können über vernetzte mobile Geräte ausgetauscht werden. Dafür können übliche Anwendung, wie beispielsweise E-Mail, WhatsApp aber auch Facebook verwendet werden. Für die Parametrierung kann bevorzugt eine Datenübertragung zwischen Mobilgerät und Vorrichtung über die Schnittstellte verwendet werden.

In einer bevorzugten Ausführungsform ist die Vorrichtung konfiguriert für eine Übertragung eines Regelprogramms der Regelvorrichtung durch das Mobilgerät. Das Regelprogramm umfasst bevorzugt einen Algorithmus, durch den bestimmt wird, auf welche Weise Behandlungstemperatur und/oder Behandlungsdauer der Vorrichtung geregelt werden. Das Regelprogramm kann beispielsweise bestimmte Regelalgorithmen umfassen. Neben Regelvorrichtungen, deren Regelalgorithmus nach einmaliger Festsetzung und mehr verändert werden kann, wie es beispielsweise bei einigen Mikroprozessoren der Fall ist, können Regelvorrichtung eingesetzt werden, welche mehrmals programmierbar sind. Das Programm kann dabei softwarebasiert und/oder hardwarebasiert sein. Beispielsweise kann ein sogenannter Field Programmable Gate Array (FPGA) als Regelvorrichtung verwendet werden, welcher durch ein übertragenes Regelprogramm komplett neu verschaltet werden kann. Dessen logische Schaltungen, die im Zusammenspiel das Regelprogramm ergeben, können dabei umprogrammiert werden. Diese Umprogrammierung kann bei Verbindung der Vorrichtung mit einem Mobilgerät über die Schnittstelle vorgenommen werden, indem ein Regelprogramm durch das Mobilgerät übertragen und auf das FPGA Modul geladen wird. So kann ein Regelprogramm regelmäßig upgedatet und verbessert werden, ohne dass dafür die gesamte Vorrichtung ausgetauscht werden müsste. Für die Übertragung eines Regelprogramms kann bevorzugt eine Datenübertragung zwischen Mobilgerät und Vorrichtung über die Schnittstellte verwendet werden.

Es kann bevorzugt sein, dass das Mobilgerät die Regelvorrichtung umfasst. Die Funktionsweise, der Aufbau und die Vorteile dieser Ausführungsform sind bereits vorstehend beschrieben worden.

In allen vorstehend genannten Ausführungsformen kann die Vorrichtung kompakt gehalten werden und beispielsweise direkt per Schnittstelle mit dem Mobilgerät, z. B. einem Smartphone, gekoppelt werden. Ein Benutzer kann in diesem Fall die Vorrichtung durch sein Smartphone nutzen, beispielsweise indem er die Vorrichtung auf das Smartphone steckt, wobei eine mechanische Verbindung bevorzugt durch die Kopplung der Schnittstellen selber bewirkt wird, und das Smartphone zur Steuerung und/oder als Griff der Vorrichtung verwendet. Bei drahtlosen Schnittstellen kann die Vorrichtung selber so geformt sein, dass sie mechanisch, beispielsweise durch Aufstecken, mit dem Mobilgerät verbindbar ist und daher auch ohne kabelgebundene Schnittstelle eine Verbindung möglich ist. Eine solche Vorrichtung ist sehr praktisch und kann äußerst klein gehalten werden. Dabei ist es von Vorteil, dass ein Benutzer sein Mobilgerät, insbesondere sein Smartphone, meistens permanent bei sich trägt und die Vorrichtung in Verbindung mit diesem nutzen kann.

Es kann aber auch sein, dass die Vorrichtung selber autark verwendet wird, beispielsweise, wenn eine eigene Regelvorrichtung vorhanden ist. Ist gleichzeitig ein eigener, kompakter Energiespeicher vorhanden, kann die Vorrichtung autark funktionieren, jedoch gleichzeitig sehr kompakt gehalten werden.

In einer bevorzugten Ausführungsform der Vorrichtung erfolgt die Stromversorgung der Vorrichtung durch das Mobilgerät und/oder der Datenaustausch mit dem Mobilgerät und/oder die Steuerung durch das Mobilgerät über das Interface drahtlos.

Sowohl zur drahtlosen Steuerung als auch zur drahtlosen Datenübertragung findet bevorzugt eine drahtlose Übertragung von Signalen über die Schnittstelle statt. Dies kann beispielsweise in Form elektromagnetischer Wellen, insbesondere durch Übertragung von Lichtsignalen geschehen. Dafür würde die Schnittstelle bevorzugt mindestens eine Empfangseinheit und/oder eine Sendeeinheit umfassen. Eine Empfangseinheit könnte beispielsweise ein geeigneter Photoempfänger wie z. B. eine Photodiode umfassen. Zusätzlich könnten andere optische Elemente wie Linsen umfasst sein, die ein Fachmann für eine optische Freistrahlübertragung vorsehen würde. Als Sender könnte beispielsweise eine geeignete Lichtquelle wie ein Laser und/oder eine (organische) LED verwendet werden. Außerdem würde ein Fachmann geeignete optische Elemente, z. B. zur Kollimation des gesendeten Lichtstrahls, verwenden. Ebenso würden bevorzugt geeignete Wandler und/oder Treiber Anwendung finden, um zwischen optischen Signalen und geeigneten internen elektrischen Signalen der Vorrichtung zu wandeln.

Auch kann ein elektromagnetisches Signal bevorzugt auf elektromagnetischen Wellen im nichtsichtbaren Spektralbereich basieren, es können beispielsweise (Nah-) Infrarotsignale, Kurzwellen, Ultrakurzwellen und/oder Dezimeterwellen verwendet werden. Auch hier würde ein Fachmann wissen, wie er die Schnittstelle mit geeigneten Sendern und/oder Empfangsantennen, Treibern und Datenwandlern sowie Verschaltungen zwischen Schnittstelle und weiteren elektrischen Elementen der Vorrichtung vornehmen müsste.

Für eine Stromversorgung der Vorrichtung durch ein Mobilgerät über das Interface wird bevorzugt Energie beispielsweise in Form von elektromagnetischen Wellen übertragen, welche dann in der Vorrichtung in nutzbare elektrische Energie umgewandelt wird und bevorzugt an die elektrischen Elemente der Vorrichtung und/oder einem Energiespeicher weitergeleitet wird. Eine drahtlose Stromversorgung wird untenstehend detailliert beschrieben.

In einer bevorzugten Ausführungsform der Vorrichtung beträgt die Behandlungstemperatur 40 °C bis 60 °C und die Größe der Behandlungsfläche zwischen 1 cm² und 18 cm². Hierdurch kann besonders zuverlässig insbesondere großflächiger Pruritus behandelt werden. In der Vergangenheit war die Fachwelt der Auffassung, dass die Behandlung großflächiger Hautleiden nicht durch Wärmeeintrag behandelt werden könnte, da sonst die gesamte, an die Haut übertragene Wärme zu groß sei. Es wurde jedoch von den Erfindern erkannt, dass bei den offenbarten Behandlungsflächen und Behandlungstemperaturen eine effektive Behandlung von großflächigem Pruritus möglich ist. Ebenso war es überraschend, dass eine Vorrichtung, welche über eine relativ große Behandlungsfläche verfügt, auf vorstehend genannte Weise mit einem Mobilgerät verbindbar ist. Hier kann auch ein etwaiger erhöhter Energiebedarf durch Stromversorgung durch ein Mobilgerät überraschend kompensiert werden.

In einer weiteren bevorzugten Ausführungsform der Vorrichtung beträgt die Behandlungstemperatur 40 °C bis 60 °C und die Größe der Behandlungsfläche ist kleiner als 1 cm² ist. Eine solche Vorrichtung ist besonders gut geeignet für eine Behandlung von Herpes, insbesondere auf der Lippe sowie von Mückenstichen. Gleichzeitig kann eine solche Vorrichtung besonders kompakt gehalten werden. Die bei der Behandlung von Mückenstichen gewonnenen Daten einer Vielzahl von Benutzern und bevorzugt einer Vielzahl von Vorrichtungen können vorteilhafterweise zur statistischen Auswertung verwendet werden. So können beispielsweise Mückenplagen festgestellt und prognostiziert werden.
In einer bevorzugten Ausführungsform ist das Mobilgerät ausgesucht aus der Gruppe umfassend PC, Laptop, Desktop-PC, Mobiltelefon, Smartphone, Tablet-Computer, Personal Digital Assistant (PDA), Notebook, Subnotebook, Smarthandy, Walkman, Discman, MP3-Player, Taschenfernseher (tragbare Fernsehgeräte), E-Book-Lesegerät, tragbares Ausgabegerät für elektronische Medien, GPS-Gerät, tragbares Schnittstellengerät der Satellitenkommunikation, Handheld, Pocket Computer (,Taschencomputer'), Mobilcomputer, Fotoapparat, Videokamera, Armbanduhr, Taschenrechner, Fernseher, MacBook, iPhone, iPad, iPod, iMac, Mac mini, Mac Pro, Smartwatch und/oder Powerbank.

Diese Mobilgeräte haben sich zur Verwendung mit der Vorrichtung als besonders praktisch herausgestellt. Insbesondere Mobilgeräte, welche eine hohe eigene Rechenleistung und eine hohe Flexibilität bezüglich ihrer Anwendung aufweisen, können in Verbindung mit der Vorrichtung ein besonders leistungsfähiges System bilden. Solche Geräte werden beispielsweise von Smartphones repräsentiert. Bei diesen können durch auf die Vorrichtung individuell zugeschnittene Anwendungsprogramme (sogenannte Apps) überraschende Vorteile erzielt werden. Beispielsweise können durch vernetzte Benutzerdaten statistisch relevante Aussagen zu erfolgversprechenden Behandlung getroffen werden, welche bei einer genügend hohen Anzahl an ausgewerteten Daten weit über das Maß der Aussagekraft einer bestimmten medizinischen Studie hinausgehen. So kann der Erfolg der Behandlung überraschend stark gesteigert werden. Ebenso können durch Vorhersagen von Insektenplagen aufgrund aus der Vergangenheit gewonnene Nutzerdaten synergistische Effekte erzielt werden, da durch die Vorrichtung nun nicht nur Insektenstiche behandelt werden können, sondern auch verhindert werden können. Auch eine verbesserte medizinische Behandlung von Juckreiz aufgrund einer Aufwertung individueller Nutzerdaten bei einem Facharzt ist möglich.

In einer bevorzugten Ausführungsform umfasst die Vorrichtung mindestens einen Energiespeicher. Energiespeicher können beispielsweise durch mindestens einen Akku, eine Batterie und/oder einen Kondensator realisiert werden. Gemeint ist bevorzugt ein elektrischer Energiespeicher. Eine Batterie ist ein Energiespeicher, bevorzugt ein elektrochemischer Energiespeicher, dessen gespeicherte Energie nach dem Verbrauch bevorzugt nicht wieder aufgefüllt werden kann. Es können Zink-Mangan-Batterien, Alkali-Mangan-Batterien, Zinkchlorid-Batterien, Zink-Kohle-Batterien, Zink-Luft-Batterien, Quecksilberoxid-Zink-Batterien, Silberoxid-Zink-Batterien, Nickel-Oxyhydroxid-Batterien, Lithiumbatterien; Lithium-Eisensulfid-Batterien, Aluminium-Luft-Batterien, Biobatterien, z. B. auf Basis Magnesium/NaCl/Eisen + Molybdän + Wolfram und/oder Edison-Lalande-Elemente verwendet werden. Batterien können auch sogenannte Knopfzellen sein.

Ein Kondensator ist bevorzugt ein elektrisches Bauteil, welches elektrische Ladung in einem elektrischen Feld speichert.

Der Energiespeicher kann insbesondere mindestens ein Akku sein. Akkus bzw. Akkumulatoren sind im Gegensatz zu Batterien bevorzugt wiederaufladbar. Akkus können beispielsweise Lithium-Ionen-Akkus, Lithium-Cobaltdioxid-Akkus, Lithium-Polymer-Akkus, Lithium-Mangan-Akkus, Lithium-Eisenphosphat-Akkus, Lithium-Eisen-Yttrium-Phosphat-Akkus, Lithium-Titanat-Akkus, Lithium-Metall-Polymer-Akkus, Lithium-Luft-Akkus, Lithium-Schwefel-Akkus, Natrium-Nickelchlorid-Hochtemperatur-Akkus, Natrium-Schwefel-Akkus, Natrium-Ionen-Akkus, Nickel-Cadmium-Akkus, Nickel-Eisen-Akkus, Nickel-Wasserstoff-Akkus, Nickel-Metallhydrid-Akkus, Nickel-Zink-Akkus, Bleiakkus, PTMA-Akkus, Rechargeable Alkaline Manganese Akkus, Zinn-Schwefel-Lithium-Akkus, Silber-Zink-Akkus, Vanadium-Redox-Akkus und/oder Zink-Brom-Akkus, Silizium-Luft-Akkus sein. Es können auch Lithium-Polymer und/oder ein Metall-hydrid-Akkus zum Einsatz kommen, welche besonders flexible und den Anwendungen angepasste Bauformen aufweisen und besonders leistungsstark sind.

Diese Ausführungsform der Vorrichtung hat den Vorteil, dass sie sowohl bei verbundenem Interface mit einem Mobilgerät, als auch autark mit elektrischer Energie versorgt ist. Insbesondere die Ausführungsform umfassend mindestens ein Akku (oder eventuell auch ein Kondensator) kann bei verbundenem Interface mit einem Mobilgerät über dieses mit elektrischer Energie versorgt und dabei aufgeladen werden. Der Akku kann dabei so dimensioniert sein, dass er für mindestens eine Anwendung auch ohne eine verbundene Schnittstelle mit dem Mobilgerät verwendbar ist. So ist die Vorrichtung für eine Verwendung nicht auf eine Verbindung mit dem Mobilgerät angewiesen und besonders flexibel.

In einer bevorzugten Ausführungsform umfasst der Akku ein Festkörperakku, bevorzugt ein Lithium - Keramik - Akku. Dieser zeichnet sich durch eine besonders hohe Sicherheit aus und ist bevorzugt auch nach einer teilweisen Zerstörung gebrauchsfähig.

In einer bevorzugten Ausführungsform ist die Vorrichtung konfiguriert für eine Stromversorgung und/oder ein Laden des Energiespeichers durch drahtlose Energieübertragung, bevorzugt über das Interface. Bei der drahtlosen Energieübertragung wird die elektrische Energie bevorzugt übertragen, ohne dass zwischen der zu ladenden Vorrichtung und dem Mobilgerät eine Verbindung über ein elektrisches Kabel besteht. Die elektrische Energie kann dabei beispielsweise durch elektromagnetische Felder übertragen werden. Dafür kann bevorzugt induktive Kopplung durch jeweils mindestens eine Spule im Mobilgerät und in der Vorrichtung verwendet werden. Es kann ebenso vorteilhaft sein, Vorrichtung und Mobilgerät durch jeweils mindestens eine Kondensatorplatte kapazitiv zu koppeln. Auch über weite Distanzen kann durch sogenannte Fernfeldübertragung bevorzugt elektrische Energie übertragen werden, die in den zwischen Sender und Empfänger transmittierten elektromagnetischen Wellen steckt. Ebenso kann durch das Mobilgerät emittiertes Licht Energie übertragen werden, die in mindestens einer Solarzelle der Vorrichtung in elektrische Spannung umgewandelt wird. Es kann auch bevorzugt sein, elektrische Energie zwischen leitenden Oberflächen des Mobilgeräts und der Vorrichtung über den zwischen diesen Oberflächen bestehenden Kontakt direkt zu übertragen. Die übertragene Energie kann dabei direkt zum Aufheizen des Heizelements oder aber bevorzugt zum Laden eines in der Vorrichtung integrierten Akkus verwendet werden.
Ein Fachmann wüsste, dass er die Schnittstelle bzw. die Vorrichtung mit hierfür geeigneten Elementen wie z. B. mindestens einer Induktionsspule, einer Kondensatorplatte, einer Antenne, einer Solarzelle, einer Photodiode, einem Laderegler und/oder einem Spannungsregler ausstatten müsste und wie er die geeigneten Elemente verschalten müsste, um die drahtlose Energieübertragung zu realisieren.

So kann Energie übertragen werden, ohne lästige Kabel zwischen Vorrichtung und Mobilgerät zu verwenden. Da auch zur Datenübertragung, zur Parametrierung, zur Übertragung eines Regelprogramms und/oder zur Steuerung eine drahtlose Schnittstelle zum Einsatz kommen kann, kann die Verwendung von Kabeln völlig vermieden werden. Als Versender bei der drahtlosen Energieübertragung kann bevorzugt auch eine mobile Vorrichtung verwendet werden, welche nur diesem Zwecke dient und direkt in eine Steckdose eingesteckt wird.

In einer bevorzugten Ausführungsform ist die Vorrichtung konfiguriert für eine Stromversorgung und/oder ein Laden des Energiespeichers durch ein Kabel, insbesondere über die Schnittstelle. In dieser Ausführungsform umfasst die Vorrichtung, insbesondere die Schnittstelle, ein Kabel bzw. einen Stecker und/oder eine Buchse zur Verbindung mit einem Kabel. Des Weiteren ist bevorzugt, die elektrische Energie innerhalb der Vorrichtung an geeignete Elemente, insbesondere an den Energiespeicher durch beispielsweise Verbindungen weitergeleitet wird. Zwischengeschaltet ist vorteilhafterweise ein Spannungsregler und/oder Ladungsregler. Einem Fachmann sind geeignete Elemente und deren Verschaltungen bekannt. Eine Übertragung von elektrischer Energie auf diese Art ist besonders robust, energieeffizient und kostengünstig.

In einer bevorzugten Ausführungsform ist ein Interface ausgesucht ist aus der Gruppe umfassend: Bluetooth, Lightning, Klinkenstecker, Koaxialstecker, Apple 30-pin dock connector, ASUS Media Bus proprietary, CAMAC, EISA, ISA, LPC, MBus, MCA, Multibus for industrial systems, NuBus oder IEEE 1196, OPTi local bus, PCI, ATA, PATA, IDE, EIDE, ATAPI, S-100 bus oder IEEE 696, SBus oder IEEE 1496, SS-50 Bus, Runway bus, GSC/HSC, Precision Bus, STEbus, STD Bus, Unibus, Q-Bus, VLB oder VL-bus, VMEbus, PC/104, PC/104-Plus, PCI-104, PCI/104-Express, PCI/104, Zorro II and Zorro III, 1-Wire, HyperTransport, I²C, PCIe, SATA, SPI bus, UNI/O, SMBus, IrDA, WLAN, ZigBee, NFC, Wibree, WiMAX, IrDA, optischer Richtfunk, eBus, USB, Micro USB, Type C und/oder FireWire. Durch Bereitstellung eines Interface ausgesucht aus dieser Gruppe ist eine hohe Flexibilität bezüglich des verwendeten Mobilgeräts und der zur Verfügung stehenden Schnittstelle gegeben. Des Weiteren haben diese Schnittstellen ihre Tauglichkeit für eine Vielzahl verschiedenster Anwendung unter Beweis gestellt. Dabei ist einem Fachmann bekannt, wie er eine solche Schnittstelle konstruieren müsste. Beispielsweise wüsste ein Fachmann, dass er für eine Lightning Schnittstelle eine geeignete Buchse und/oder einen geeigneten Stecker verbauen müsste, ebenso wüsste er, dass er geeignete Prozessoren, z. B. Host-Controller verwenden müsste. Ihm wäre ebenso bekannt, wie er die elektrischen Elemente der Vorrichtung mit der Schnittstelle verschalten müsste.

Bei z. B. einer WLAN Schnittstelle wüsste der Fachmann ebenso, dass er mindestens eine geeignete Antenne zum Versenden und/oder Empfangen von Signalen in die Vorrichtung verbauen müsste. Auch die Auswahl geeigneter Steuerungsprozessoren, Datenwandler und/oder Controller sowie eine Verschaltung der Schnittstelle innerhalb der Vorrichtung ist für den Fachmann routinemäßig durchführbar.

Dabei kann es bevorzugt sein, die Schnittstelle zu verwenden, um die Vorrichtung zur Stromversorgung und/oder zum Aufladen direkt mit einer Steckdose zu verbinden. In einer bevorzugten Ausführungsform ist es beispielsweise möglich, mittels handelsüblichem USB-Ladegerät elektrische Energie direkt aus der Steckdose zu beziehen.

In einer bevorzugten Ausführungsform umfasst die Vorrichtung mindestens einen ersten Temperatursensor zur Messung der Temperatur der Behandlungsfläche und die Regelvorrichtung und/oder Steuerung stellt basierend auf den Messdaten des Temperatursensors die Temperatur des mindestens einen Heizelementes ein.

Im Sinne der Erfindung ist ein Temperatursensor bevorzugt ein elektrisches oder elektronisches Bauelement, welches in Abhängigkeit der Temperatur am Sensor ein elektrisches Signal generiert. Im Stand der Technik sind eine Vielzahl von Temperatursensoren bekannt, wie beispielsweise Halbleiter-Temperatursensoren, Widerstandstemperatursensoren, pyroelektrische Materialien, Thermoelemente oder Schwingquarze. Die Regelvorrichtung ist weiterhin bevorzugt derart konfiguriert, dass diese die Messwerte der Temperatursensoren aufnehmen und auswerten kann, um eine Regulation der Heizplatten zu bewirken. Die Regulation der Heizplatten kann bevorzugt mithilfe des Anliegens eines elektrischen Stroms oder einer Spannung erfolgen. Es ist besonders bevorzugt, dass der Temperatursensor die Temperatur der Behandlungsfläche direkt misst, d.h. dass der Temperatursensor in Kontakt mit der Behandlungsfläche ist, wobei sich der Temperatursensor sowohl auf der inneren Seite der Behandlungsfläche, als auch auf der äußeren Seite der Behandlungsfläche befinden kann oder aber in die Behandlungsfläche implementiert ist. Es kann aber auch bevorzugt sein, dass der Temperatursensor nicht die Behandlungsfläche direkt, sondern die Heizelemente oder einen Materialpunkt zwischen den Heizelementen und der Behandlungsfläche kontaktiert und überwacht. Im Falle von mehreren Heizelementen, welche die Behandlungsfläche erhitzen, kann es beispielsweise auch bevorzugt sein den Temperatursensor zwischen den Heizelementen zu platzieren. Aus den Messdaten für die Temperatur über die Heizelemente oder einem Messpunkt in einer bestimmten Distanz zur Behandlungsfläche kann ebenfalls auf die Temperatur der Behandlungsfläche geschlossen werden. Im Sinne der Erfindung ist es bevorzugt, dass die Temperatur der Behandlungsfläche die durchschnittliche Temperatur der Behandlungsfläche meint.

Eine Auswertung der Temperatur der Behandlungsfläche erlaubt eine besonders präzise Regulation des mindestens einen Heizelementes, um eine optimale Temperaturverteilung auf der Behandlungsfläche und somit Wärmeübertragung an die zu behandelnden Hautpartien sicherzustellen. Insbesondere im Hinblick auf die vielfältigen Anwendungsmöglichkeiten der Vorrichtung zur Behandlung verschiedener Erkrankungen, welche mit Juckreiz einhergehen können, ist eine temperaturbasierte Feedback-Regulation mit Hilfe der Regelvorrichtung geeignet, um eine zuverlässige hyperthermische Behandlung mit optimalen Temperaturwerten durchzuführen. Eine solche Vorrichtung zur Steuerung der Behandlungstemperatur ist besonders einfach, robusten und kostengünstig.

In einer bevorzugten Ausführungsform heizt das Heizelement die Behandlungsfläche auf eine Behandlungstemperatur von 40 °C - 65°C auf. Die Bereitstellung einer Behandlungstemperatur von 40 °C bis 65 °C stellt eine überraschend verbesserte Behandlung von Juckreiz dar.

In einer bevorzugten Ausführungsform heizt das Heizelement die Behandlungsfläche auf eine Behandlungstemperatur von 50 bis 65°C, vorzugsweise 55 bis 60°C, auf. Es gab bezüglich der Behandlungstemperaturen von Juckreiz Fehlvorstellungen der Fachwelt, die mit der bereitgestellten Behandlungstemperatur überwunden werden konnten. Auch war es überraschend, dass durch eine Schnittstelle mit einem Mobilgerät ausreichend Energie für diese bevorzugte Behandlungstemperatur bereitgestellt werden konnte.

In einer bevorzugten Ausführungsform heizt das Heizelement die Behandlungsfläche auf eine Behandlungstemperatur von 42 °C - 56°C auf. Die Leistungen bezüglich der Behandlung von Juckreiz konnte durch den in dieser Ausführungsform bereitgestellten Temperaturbereich überraschend gesteigert werden.

In einer bevorzugten Ausführungsform heizt das Heizelement die Behandlungsfläche auf eine Behandlungstemperatur von 42 °C - 53°C auf. So kann ein weiteres Mittel zur Behandlung von Juckreiz bereitgestellt werden.

In einer bevorzugten Ausführungsform heizt das Heizelement die Behandlungsfläche auf eine Behandlungstemperatur von 43 °C - 47°C auf. Die Effektivität dieser Behandlungstemperaturen bei der Behandlung von Juckreiz ist überraschend groß. Gleichzeitig ist auch die elektrische Effektivität bezüglich des Energieverbrauchs größer als in anderen Temperaturbereichen. Hierdurch kann die Gesamteffektivität synergistisch gesteigert werden.

In einer bevorzugten Ausführungsform heizt das Heizelement die Behandlungsfläche auf eine Behandlungstemperatur von 50 °C - 53°C auf. Dieser Temperaturbereich hat sich als besonders wirksam und bei Probanden als beliebt herausgestellt, sodass ein großer wirtschaftlicher Erfolg absehbar ist.

In einer bevorzugten Ausführungsform heizt das Heizelement die Behandlungsfläche auf eine Behandlungstemperatur von 50 °C - 55 °C auf. In diesem Temperaturbereich ist die Ausbeute an erzeugter Wärme pro verbrauchte Energie besonders hoch.

In einer bevorzugten Ausführungsform heizt das Heizelement die Behandlungsfläche auf eine Behandlungstemperatur von 55 °C - 60 °C auf. Die Behandlungstemperatur in diesem Bereich ist überraschend wirksam. Hier kam es zu einem glücklichen Griff, denn aus einem weiten Parameterbereich wurde gerade der Bereich herausgegriffen, der unerwarteterweise besonders erfolgreich gegen Juckreiz wirkte.

In einer bevorzugten Ausführungsform heizt das Heizelement die Behandlungsfläche auf eine Behandlungstemperatur von 60 °C - 65 °C auf. Dieser Temperaturbereich wurde entgegen dem Trend in der wissenschaftlichen Technik gewählt.

In einer bevorzugten Ausführungsform wird die Behandlungstemperatur für eine Behandlungsdauer von 1 - 60 sec aufrechterhalten. So können die technischen Möglichkeiten zur Behandlung von Juckreiz durch Wärme erhöht werden.

In einer bevorzugten Ausführungsform wird die Behandlungstemperatur für eine Behandlungsdauer von 1 - 10 sec aufrechterhalten. Eine solche kurze Behandlungsdauer bedeutet eine überraschende Ersparnis an Zeit bei der Behandlung von Juckreiz.

In einer bevorzugten Ausführungsform wird die Behandlungstemperatur für eine Behandlungsdauer von 10 - 20 sec aufrechterhalten. Diese Behandlungsdauer hat sich bei der Behandlung von Juckreiz als besonders zuverlässig erwiesen.

In einer bevorzugten Ausführungsform wird die Behandlungstemperatur für eine Behandlungsdauer von 20 - 30 sec aufrechterhalten. So kann überraschenderweise ein zweiter Weg zur Behandlung von Juckreiz durch Wärme bereitgestellt werden.

In einer bevorzugten Ausführungsform wird die Behandlungstemperatur für eine Behandlungsdauer von 30 - 40 sec aufrechterhalten. Es hat sich gezeigt, dass bei einer solchen Behandlungsdauer die Ausbeute der abgegebenen Wärmemenge besonders hoch ist.

In einer bevorzugten Ausführungsform wird die Behandlungstemperatur für eine Behandlungsdauer von 40 - 50 sec aufrechterhalten. Es gab in der Fachwelt Fehlvorstellungen über die geeignete Behandlungsdauer für die hyperthermische Behandlung von Juckreiz, welche durch diese Behandlungsdauer überwunden wurden.

In einer bevorzugten Ausführungsform wird die Behandlungstemperatur für eine Behandlungsdauer von 50 - 60 sec aufrechterhalten. Es können so überraschend Fehler bei der Behandlung von Juckreiz vermieden werden.

In einer bevorzugten Ausführungsform wird die Behandlungstemperatur für eine Behandlungsdauer von 2 - 20 sec aufrechterhalten. Diese Behandlungsdauer stellt eine entscheidende Verbesserung gegenüber bekannten Verfahren zur Behandlung von Juckreiz dar.

In einer bevorzugten Ausführungsform wird die Behandlungstemperatur für eine Behandlungsdauer von 2 - 12 sec aufrechterhalten. Aus einer Vielzahl vorstellbarer oder bekannter Behandlungsdauern wurde durch einen glücklichen Griff diese Behandlungsdauer ausgewählt, welche überraschend effektiv bei der Behandlung von Juckreiz ist. Dies war nicht voraussehbar.

In einer bevorzugten Ausführungsform wird die Behandlungstemperatur für eine Behandlungsdauer von 3 bis 6 sec aufrechterhalten. Durch diese kurze und gleichzeitig besonders wirksame Behandlungsdauer kann die Effizienz der Vorrichtung gesteigert werden.

In einer bevorzugten Ausführungsform wird die Behandlungstemperatur für eine Behandlungsdauer von 4 - 6 sec aufrechterhalten. Diese Behandlungsdauer stellt eine entscheidende Abkehr vom technisch Üblichen dar.

In einer bevorzugten Ausführungsform heizt das Heizelement die Behandlungsfläche auf eine Behandlungstemperatur von 50 bis 65°C, vorzugsweise 55 bis 60°C, auf und die Behandlungstemperatur wird für eine Behandlungsdauer von 2 bis 12 sec, vorzugsweise 3 bis 6 sec, aufrechterhalten. Tests haben ergeben, dass, wenn die Behandlung unmittelbar nach dem Stich erfolgt, bei einer Maximaltemperatur von 55 °C Mückenstiche erfolgreich inaktiviert werden können. Die Einwirkdauer ist bei einer Temperatur von 55 °C unkritisch, so dass die Behandlung bei dieser Temperatur im Bedarfsfall mehrfach wiederholt werden kann. Darüber hinaus wird infolge der betäubenden Wirkung von Insektengift eine Wärmebehandlung der Haut an der Einstichstelle als deutlich weniger unangenehm empfunden als in anderen Bereichen.

In einer bevorzugten Ausführungsform heizt das Heizelement die Behandlungsfläche auf eine Behandlungstemperatur zwischen 42 °C und 56 °C auf und die Behandlungstemperatur wird für eine Behandlungsdauer von 2 sec bis 20 sec aufrechterhalten.

Hierdurch können Schmerzen und weitere unangenehme Empfindungen wie bspw. ein Ziehen oder Juckreiz überraschend wirksam gelindert werden. Der Wärmeimpuls bewirkt eine Nervenstimulation, welche die subjektive Wahrnehmung von Schmerzen oder Juckreiz an den betreffenden Stellen stark reduziert. Die Wärmeübertragung führt überraschend zu einer Überlagerung der unangenehmen Empfindung durch andere temperaturabhängige Hautempfindungen. Entgegen konventionellen Methoden zur Behandlung von Herpes wird dabei zusätzlich eine Regulation der Schmerzrezeptoren anvisiert und durch die bevorzugte Wärmebehandlung die freien Nervenenden der C-Fasern aktiviert. Insbesondere wird die Erkenntnis über den Wirkmechanismus bei Herpes ausgenutzt, um durch eine Wärmebehandlung in überraschender Weise die Sinneswahrnehmung, welche durch die Fasern ausgelöst werden, zu regulieren. Auch ein Einsatz der Vorrichtung zur Behandlung von Herpes erlauben die genannten Zeiträume und Temperaturparameter auf überraschende Weise. Ebenso sind Juckreizerkrankungen z.B. nach Kontakt mit giftigen Nesseltieren oder Pflanzen, wie Brennnesseln oder bei Insektenstichen und -bissen, behandelbar. Es können sogar thermolabile Gifte von Insektenstichen neutralisiert werden.

Es wurde darüber hinaus erkannt, dass bei dieser Kombination eine besonders starke Überlagerung der unangenehmen Empfindungen erreicht werden kann, da in den betreffenden Hautpartien lokal die Thermo- und Capsaicinrezeptoren TRPV1 und TRPV2 zeitgleich aktiviert werden. Ein Fachmann würde auch unter Kenntnis der Literatur nicht davon ausgehen, dass gerade die Aktivierung dieser Rezeptoren eine besonders effektive Überlagerung der unangenehmen Empfindungen ermöglicht, welche auch bei der Behandlung von Herpes funktioniert. Dies ist eine überraschende Erkenntnis.

Durch diese Kombination von Behandlungstemperatur und Behandlungsdauer können außerdem die technischen Möglichkeiten zur Behandlung von Juckreiz erhöht werden.

In einer bevorzugten Ausführungsform heizt das Heizelement die Behandlungsfläche auf eine Behandlungstemperatur von 49 °C bis 53 °C auf und die Behandlungstemperatur wird für eine Behandlungsdauer von 3 bis 20 sec aufrechterhalten. Bevorzugte Behandlungstemperatur und - dauer sind auf überraschende Weise geeignet, durch Herpesviren verursachte Herpeserkrankungen der Haut in Form von mit Bläschenbildung einhergehendem Hautausschlag, angrenzende Hautbereiche oder auf durch erste Anzeichen des Herpes erkennbare erkrankte Hautbereiche zu behandeln, ohne dass es gleichzeitig zu schmerzhaften Empfindungen oder gar Verbrennungen kommt. Dies ist insbesondere im empfindlichen Bereich der Mundpartie, vor allem der Lippen, von besonderer Bedeutung. Auch bei der Behandlung von Juckreiz kann durch die genannte Kombination eine überraschende Leistungssteigerung der Behandlungseffektivität erreicht werden.

In einer bevorzugten Ausführungsform heizt das Heizelement die Behandlungsfläche auf eine Behandlungstemperatur zwischen 40°C und 65°C auf und die Behandlungstemperatur wird für eine Behandlungsdauer von 4 sec bis 6 sec aufrechterhalten.

Durch die Regulation der Behandlungsfläche auf einer Temperatur zwischen 40°C und 65°C für eine Behandlungsphase zwischen 2 s und 12 s, bevorzugt zwischen 4s und 12 s, besonders bevorzugt zwischen 4 s - 6 s, wird ein Hitzeimpuls generiert, welcher es erlaubt eine wohldefinierte Wärmemenge auf die Hautstelle auf kontrollierte Weise zu bringen. Auf diese Weise kann eine effektive Behandlung unter Ersparnis an Zeit bereitgestellt werden.

In einer bevorzugten Ausführungsform heizt das Heizelement die Behandlungsfläche auf eine Behandlungstemperatur zwischen 42°C und 53°C, besonders bevorzugt zwischen 50°C und 53°C auf und die Behandlungstemperatur wird für eine Behandlungsdauer von 4 sec bis 6 sec aufrechterhalten. Es hat sich völlig überraschend gezeigt, dass mit den vorgenannten Parametern ein Juckreiz insbesondere auf großflächigen Hautpartien besonders stark reduziert werden kann. Eine Kombination einer Behandlungstemperatur von 42°C und 53°C und insbesondere mit der bevorzugten Behandlungstemperatur zwischen 50°C und 53°C erlaubt eine Wirkung auf die Hautpartien, welche den Juckreiz schnell und effektiv lindern. Es wurde erkannt, dass eine besonders starke Überlagerung des Juckempfindens erreicht werden kann, da in den betreffenden Hautpartien lokal die Thermo- und Capsaicinrezeptoren TRPV1 und TRPV2 zeitgleich aktiviert werden.

Die bevorzugt genannte Ausführungsform, welche eine Temperaturregelung der Behandlungsfläche in einem engen Bereich zwischen 50°C und 53°C vorsieht, erlaubt auf überraschend effektive Weise eine zeitgleiche Aktivierung der Rezeptoren, ohne unangenehm starke Schmerzempfindungen bei den behandelten Personen auszulösen. Durch experimentelle Versuche konnte der Bereich der Aktivierungsschwelle des TRPV2 als besonders optimierter Wirkbereich ermittelt werden. Hierbei kommt es voraussichtlich zu einem Feedbackmechanismus zwischen den Rezeptoren, welcher den Juckreiz besonders effektiv überlagert, ohne Nebenwirkungen zu verursachen. Ein Fachmann konnte dies nicht erwarten. Vielmehr wäre ein Fachmann davon ausgegangen, dass es bei einer Behandlungstemperatur von 50°C - 53°C über einen Zeitraum von 4 s bis 6 s zu starken Hautirritationen oder Schmerzen bis hin zu leichten Verbrennungen kommt. Stattdessen führt die bevorzugte Behandlung der Hautpartien zu einem Abmildern des Juckempfindens, welches überraschenderweise noch Stunden nach der Behandlung anhält. Die langanhaltende Wirkungsweise der bevorzugten Ausführungsform ist zu mindestens teilweise auf eine Immunregulation durch die Wärmübertragung zurückzuführen. So wird nicht nur das Schmerzempfinden überlagert, sondern durch eine Regulation des Immunsystems wird die lokale Reizung der Haut aktiv unterdrückt. Vorteilhafterweise kann daher eine einmalige Behandlung bereits zu einem nachhaltigen Nachlassen des Juckempfindens führen. Es kann aber auch bevorzugt sein, mehrmals in zeitlicher Abfolge eine Behandlung durchzuführen. Die intervallartige Übertragung der Wärme mit einer Behandlungsphase von 4 s bis 6 s erreicht eine optimale Wirkung auf die Signalwege des Pruritus ohne unerwünschte Nebenwirkungen auszulösen. Es hat sich überraschenderweise besonders für die bevorzugte Behandlungsdauer von 4s - 6s und die vorgesehene Behandlungstemperatur, insbesondere für die besonders bevorzugte Behandlungstemperatur von 50 °C - 53 °C herausgestellt, dass eine außergewöhnlich wirkungsvolle Aktivierung der TRPV1 und insbesondere der TRPV2 Rezeptoren stattfindet. Es war völlig überraschend, dass diese spezifische Auswahl von Behandlungstemperatur und Behandlungsdauer besonders effektiv zu einem Nachlassen des Juckreizes beiträgt. Auch die C-Fasern, insbesondere deren freie Enden, werden durch diese bevorzugte Auswahl ideal angesprochen.

In einer bevorzugten Ausführungsform beträgt die Größe der Behandlungsfläche zwischen 0,1 cm² bis 18 cm². So können die technischen Möglichkeiten zur Behandlung von Juckreiz vermehrt werden.

In einer bevorzugten Ausführungsform beträgt die Größe der Behandlungsfläche zwischen 1 cm² bis 18 cm². Mit einer Behandlungsfläche dieser Größe können auch in kurzer Zeit Behandlungserfolge bei der Behandlung von Juckreiz erzielt werden.

In einer bevorzugten Ausführungsform beträgt die Größe der Behandlungsfläche mindestens 6 cm², bevorzugt mindestens 7 cm².

Äußere Reize chemischer, mechanischer oder physikalischer Natur, die Juckempfinden auslösen können, werden von drei unterschiedlichen Rezeptorzellen (Sinneszellen) wahrgenommen. Bei diesen Sinneszellen handelt es sich um so genannte offene Nervenendigungen, deren reizaufnehmende Strukturen in der Epidermis und der darunterliegenden Dermis lokalisiert sind, und deren Axone die Signale über wahrgenommene Reize ins Rückenmark weiterleiten. Bei diesen Sinneszellen sind die unmyelenisierten C-Fasern von besonderer Bedeutung. Deren rezeptive Strukturen befinden sich teilweise bis 0.1 mm unter der Hautoberfläche. Bei den C-Fasern unterscheidet man polymodale mechanisch und hitzesensitive Fasern und mechanisch insensitive C-Fasern, die aber auch durch Hitze stimuliert werden können. C-Fasern nehmen nicht nur pruritogene Reize war, sondern dienen auch als Nozizeptoren (Schmerzrezeptoren). In der Literatur wurde gezeigt, dass Hitzereize als Gegenreiz Juckempfinden unterdrücken können. Die einzelnen C-Fasern nehmen Reize eines bestimmten Areals der Haut wahr, wobei ein definiertes Hautareal von einer Sinneszelle innerviert wird. Diese Region wird als rezeptives Feld bezeichnet. Die rezeptiven Felder von C-Fasern können teilweise überlappend sein. Durch Untersuchungen am Menschen durch so genanntes Micromapping wurde überraschenderweise erkannt, dass die mechanisch insensitiven C-Fasern rezeptive Felder von bis zu 5 cm² Größe haben; die von mechanisch sensitiven C-Fasern sind etwas kleiner und bis zu 2 cm² groß. Überraschenderweise hat sich gezeigt, dass ab einer bevorzugten Größe der Behandlungsfläche von ungefähr 7 cm² erreicht werden kann, die rezeptiven Felder beider Typen von C-Fasern besonders gut anzusprechen. Bei der bevorzugten Behandlungsgröße zwischen 7 cm² und 18 cm² werden somit die rezeptiven Felder der unterschiedlichen C-Fasertypen überraschend gut abgedeckt und darüber hinaus wird der Effekt der horizontal abfließenden Wärme kompensiert. Somit kann ein Juckreiz, auch von betroffenen Hautpartien kleinerer Größe als der Behandlungsfläche, überraschender Weise sehr effektiv behandelt werden. Hier ging die Fachwelt von völlig anderen Vorstellung aus, die Benutzung solch großer Behandlungsflächen wurden als nicht realisierbar betrachtet.

In einer bevorzugten Ausführungsform beträgt die Größe der Behandlungsfläche weniger als 1 cm². So wurde ein neues Anwendungsgebiet für Vorrichtungen zur Behandlung von Juckreiz eröffnet.

In einer bevorzugten Ausführungsform beträgt die Größe der Behandlungsfläche weniger als 40 mm². Insbesondere bei Herpeserkrankungen, vor allem am Mund (sogenanntes Herpes labialis), ist die bevorzugte maximale Größe der Behandlungsfläche ideal, um alle möglichen betroffenen Stellen abzudecken. Insbesondere eine Behandlungsfläche von 20 mm² ist geeignet, alle typischen betroffenen Hautpartien bei nur einmaligem Auflegen der Vorrichtung abzudecken. Des Weiteren kann eine Herpesbehandlungseinrichtung, welche eine solche Behandlungsfläche aufweist, besonders kompakt gehalten werden. So können Vorrichtungsgrößen, welche der eines Lippenstifts entsprechen, erreicht werden. Ein solch kompaktes Gerät wird gern und bereitwillig dauerhaft am Körper oder in einer mitgeführten Tasche getragen, sodass eine Behandlung jederzeit vorgenommen werden kann. Dies steigert den Behandlungserfolg erheblich. Die Behandlungsfläche ist bevorzugt rund, dies eignet sich besonders zur Behandlung von Herpes, dessen betroffene Hautpartien oftmals organische, annähernd runde Form aufweisen.

In einer bevorzugten Ausführungsform heizt das Heizelement die Behandlungsfläche auf eine Behandlungstemperatur von 50 bis 65°C, vorzugsweise 55 bis 60°C, auf, wobei die Behandlungstemperatur für eine Behandlungsdauer von 2 bis 12 sec, vorzugsweise 3 bis 6 sec, aufrechterhalten wird und wobei die Größe der Behandlungsfläche zwischen 1 cm² bis 18 cm² beträgt. Hier wurden mehrere an sich bekannte Elemente zu einer Kombination zusammengeführt, die den überraschenden Effekt aufweist, dass auch die hohen Behandlungstemperaturen und großen Behandlungsflächen überraschend geringe Schmerzen bzw. unangenehmen Empfindungen verursacht.

In einer bevorzugten Ausführungsform heizt das Heizelement die Behandlungsfläche auf eine Behandlungstemperatur von 50 bis 65°C, vorzugsweise 55 bis 60°C, auf, wobei die Behandlungstemperatur für eine Behandlungsdauer von 2 bis 12 sec, vorzugsweise 3 bis 6 sec, aufrechterhalten wird und wobei die Größe der Behandlungsfläche mindestens 6 cm², bevorzugt mindestens 7 cm² beträgt. Die Aussagen der Fachliteratur bezüglich einer Kombination von Behandlungstemperaturen, Behandlungsdauer und Behandlungsfläche wiesen irrtümlicherweise bislang in eine andere Richtung.

In einer bevorzugten Ausführungsform heizt das Heizelement die Behandlungsfläche auf eine Behandlungstemperatur von 50 bis 65°C, vorzugsweise 55 bis 60°C, auf, wobei die Behandlungstemperatur für eine Behandlungsdauer von 2 bis 12 sec, vorzugsweise 3 bis 6 sec, aufrechterhalten wird und wobei die Größe der Behandlungsfläche weniger als 1 cm² beträgt. Diese Kombination von Behandlung Wesentlichen Größen vergrößert die Effektivität der Vorrichtung für die Behandlung von Juckreiz.

In einer bevorzugten Ausführungsform heizt das Heizelement die Behandlungsfläche auf eine Behandlungstemperatur von 50 bis 65°C, vorzugsweise 55 bis 60°C, auf, wobei die Behandlungstemperatur für eine Behandlungsdauer von 2 bis 12 sec, vorzugsweise 3 bis 6 sec, aufrechterhalten wird und wobei die Größe der Behandlungsfläche weniger als 40 mm² beträgt. So kann eine gleichzeitig kompakte und effektive Vorrichtung zur Behandlung von Juckreiz bereitgestellt werden.

In einer bevorzugten Ausführungsform heizt das Heizelement die Behandlungsfläche auf eine Behandlungstemperatur zwischen 42 °C und 56 °C auf, wobei die Behandlungstemperatur für eine Behandlungsdauer von 2 sec bis 20 sec aufrechterhalten wird und wobei die Größe der Behandlungsfläche zwischen 1 cm² bis 18 cm² beträgt. Eine solche Vorrichtung zur Behandlung von Juckreiz ist besonders zuverlässig im Gebrauch.

In einer bevorzugten Ausführungsform heizt das Heizelement die Behandlungsfläche auf eine Behandlungstemperatur zwischen 42 °C und 56 °C auf, wobei die Behandlungstemperatur für eine Behandlungsdauer von 2 sec bis 20 sec aufrechterhalten wird und wobei die Größe der Behandlungsfläche mindestens 6 cm², bevorzugt mindestens 7 cm² beträgt. Die bisherige Entwicklung von Vorrichtungen zur Hautbehandlung ging in eine völlig andere Richtung und legt diese Kombination nicht nahe. Dabei kann diese Kombination von Behandlungstemperatur, Behandlungsdauer und Behandlungsfläche auf synergistische Weise die Effektivität der Behandlung erhöhen, und zwar auf eine Weise, die größer ist, als es die bekannten Behandlungseffektivitäten der jeweiligen Parameter einzeln erwarten ließen.

In einer bevorzugten Ausführungsform heizt das Heizelement die Behandlungsfläche auf eine Behandlungstemperatur zwischen 42 °C und 56 °C auf, wobei die Behandlungstemperatur für eine Behandlungsdauer von 2 sec bis 20 sec aufrechterhalten wird und wobei die Größe der Behandlungsfläche weniger als 1 cm² beträgt. Diese Kombination war ein glücklicher Griff der Erfinder, da aus einer Vielzahl möglicher Kombinationen der beschriebenen Größen diejenige ausgesucht wurde, die einen unerwarteten Behandlungserfolg bewirkte.

In einer bevorzugten Ausführungsform heizt das Heizelement die Behandlungsfläche auf eine Behandlungstemperatur zwischen 42 °C und 56 °C auf, wobei die Behandlungstemperatur für eine Behandlungsdauer von 2 sec bis 20 sec aufrechterhalten wird und wobei die Größe der Behandlungsfläche weniger als 40 mm² beträgt. Diese Kombination stellt eine Abkehr vom technisch üblichen dar.

In einer bevorzugten Ausführungsform heizt das Heizelement die Behandlungsfläche auf eine Behandlungstemperatur von 49 °C bis 53 °C auf, wobei die Behandlungstemperatur für eine Behandlungsdauer von 3 bis 20 sec aufrechterhalten wird und wobei die Größe der Behandlungsfläche zwischen 1 cm² bis 18 cm² beträgt.

Die Zusammenführung der beschriebenen Größen in dieser Ausführungsform führen überraschenderweise dazu, dass mehrere, auch weit voneinander entfernte Hautleiden, wie beispielsweise Insektenstiche, zeitgleich behandelt werden können. Auch Hautleiden, die eine größere Fläche als die Behandlungsfläche abdecken, können während einer einzigen Behandlung mit diesen Parametern effektiv gelindert werden.

In einer bevorzugten Ausführungsform heizt das Heizelement die Behandlungsfläche auf eine Behandlungstemperatur von 49 °C bis 53 °C auf, wobei die Behandlungstemperatur für eine Behandlungsdauer von 3 bis 20 sec aufrechterhalten wird und wobei die Größe der Behandlungsfläche mindestens 6 cm², bevorzugt mindestens 7 cm² beträgt.
In der Fachliteratur ging man hingegen davon aus, dass eine so große Behandlungsfläche bei den offenbarten Behandlungsdauern und Behandlungstemperaturen nicht wirksam sei.

In einer bevorzugten Ausführungsform heizt das Heizelement die Behandlungsfläche auf eine Behandlungstemperatur von 49 °C bis 53 °C auf, wobei die Behandlungstemperatur für eine Behandlungsdauer von 3 bis 20 sec aufrechterhalten wird und wobei die Größe der Behandlungsfläche weniger als 1 cm² beträgt. Durch diese Kombination der beschriebenen Größen kann eine überraschende Miniaturisierung der Vorrichtung erreicht werden.

In einer bevorzugten Ausführungsform heizt das Heizelement die Behandlungsfläche auf eine Behandlungstemperatur von 49 °C bis 53 °C auf, wobei die Behandlungstemperatur für eine Behandlungsdauer von 3 bis 20 sec aufrechterhalten wird und wobei die Größe der Behandlungsfläche weniger als 40 mm² beträgt. So kann ein weiteres Mittel zur Behandlung von Juckreiz und insbesondere auch Herpes bereitgestellt werden.

In einer bevorzugten Ausführungsform heizt das Heizelement die Behandlungsfläche auf eine Behandlungstemperatur zwischen 40°C und 65°C auf, wobei die Behandlungstemperatur für eine Behandlungsdauer von 4 sec bis 6 sec aufrechterhalten wird und wobei die Größe der Behandlungsfläche zwischen 1 cm² bis 18 cm² beträgt. Durch die Kombination relevanter Größen kann eine besonders hohe Ausbeute der in die Haut eingebrachten Wärmemenge erzielt werden.

In einer bevorzugten Ausführungsform heizt das Heizelement die Behandlungsfläche auf eine Behandlungstemperatur zwischen 40°C und 65°C auf, wobei die Behandlungstemperatur für eine Behandlungsdauer von 4 sec bis 6 sec aufrechterhalten wird und wobei die Größe der Behandlungsfläche mindestens 6 cm², bevorzugt mindestens 7 cm² beträgt. Durch diese Parameterkombination kann eine besonders zuverlässige Behandlung erreicht werden.

In einer bevorzugten Ausführungsform heizt das Heizelement die Behandlungsfläche auf eine Behandlungstemperatur zwischen 40°C und 65°C auf, wobei die Behandlungstemperatur für eine Behandlungsdauer von 4 sec bis 6 sec aufrechterhalten wird und wobei die Größe der Behandlungsfläche weniger als 1 cm² beträgt.

Diese Kombination relevanter Größen war ein glücklicher Griff der Erfinder. Viele mögliche Kombination standen zur Auswahl, wobei die außergewöhnliche Verbesserung einer Behandlung von Juckreiz gerade dieser Kombination nicht zu erwarten war.

In einer bevorzugten Ausführungsform heizt das Heizelement die Behandlungsfläche auf eine Behandlungstemperatur zwischen 40°C und 65°C auf, wobei die Behandlungstemperatur für eine Behandlungsdauer von 4 sec bis 6 sec aufrechterhalten wird und wobei die Größe der Behandlungsfläche weniger als 40 mm² beträgt. Durch den hier beschriebenen großen Temperaturbereich und die kleine Behandlungsfläche in Kombination mit einer Behandlungsdauer von 4 bis 6 Sekunden können sehr flexibel verschiedenste Leiden der Haut, welche Juckreiz verursachen, behandelt werden.

In einer bevorzugten Ausführungsform heizt das Heizelement die Behandlungsfläche auf eine Behandlungstemperatur zwischen 42°C und 53°C, besonders bevorzugt zwischen 50°C und 53°C auf, wobei die Behandlungstemperatur für eine Behandlungsdauer von 4 sec bis 6 sec aufrechterhalten wird und wobei die Größe der Behandlungsfläche zwischen 1 cm² bis 18 cm² beträgt. Eine Vorrichtung, welche diese Kombination aufweist, hat sich als überraschend wartungsarmen und robust herausgestellt. Auch unter widrigen Bedingungen, bei beispielsweise hoher Luftfeuchtigkeit und großer Hitze, funktioniert die Vorrichtung überraschend einwandfrei.

In einer bevorzugten Ausführungsform heizt das Heizelement die Behandlungsfläche auf eine Behandlungstemperatur zwischen 42°C und 53°C, besonders bevorzugt zwischen 50°C und 53°C auf, wobei die Behandlungstemperatur für eine Behandlungsdauer von 4 sec bis 6 sec aufrechterhalten wird und wobei die Größe der Behandlungsfläche mindestens 6 cm², bevorzugt mindestens 7 cm² beträgt. Hier ist die Effektivität der Behandlung besonders hoch.

In einer bevorzugten Ausführungsform heizt das Heizelement die Behandlungsfläche auf eine Behandlungstemperatur zwischen 42°C und 53°C, besonders bevorzugt zwischen 50°C und 53°C auf, wobei die Behandlungstemperatur für eine Behandlungsdauer von 4 sec bis 6 sec aufrechterhalten wird und wobei die Größe der Behandlungsfläche weniger als 1 cm² beträgt. Eine solche Vorrichtung weist eine hohe Energieausbeute auf. Auch leistungsschwache Stromquellen genügen, um eine effektive Behandlung durchzuführen.

In einer bevorzugten Ausführungsform heizt das Heizelement die Behandlungsfläche auf eine Behandlungstemperatur zwischen 42°C und 53°C, besonders bevorzugt zwischen 50°C und 53°C auf, wobei die Behandlungstemperatur für eine Behandlungsdauer von 4 sec bis 6 sec aufrechterhalten wird und wobei die Größe der Behandlungsfläche weniger als 40 mm² beträgt. So kann eine weitere Alternative zur Behandlung von Juckreiz bereitgestellt werden.

In einer bevorzugten Ausführungsform begrenzt ein hardwareimplementierter Temperaturwächter die Maximaltemperatur der Behandlungsfläche reversibel und eine Schmelzsicherung schaltet bei Kurzschluss oder ungeregeltem Durchheizen die Vorrichtung ab. Die Maximaltemperatur bezeichnet bevorzugt jene Temperatur, welche die Behandlungsfläche maximal während der Behandlungsphase erreicht. Der hardwareimplementierte Temperaturwächter erlaubt vorteilhafterweise eine Sicherstellung, dass die Maximaltemperatur nicht überschritten wird. Im Sinne der Erfindung bezeichnen Angaben wie ungefähr, ca., nahezu oder synonyme Begriffe bevorzugt eine Toleranzspanne von weniger als ± 10%, bevorzugt weniger als ± 5% besonders bevorzugt weniger als ± 1%. Im Sinne der Erfindung bezeichnet ein "hardwareimplementierte Temperaturwächter" bevorzugt ein Temperaturkontrollsystem für die Behandlungsoberfläche, welches hardwarebasiert die Stromversorgung der Heizelemente für die Behandlungsfläche abschalten kann. Insbesondere erlaubt der "hardwareimplementierte Temperaturwächter" bevorzugt, dass eine Abschaltung der Stromversorgung der Heizelemente bei Überschreitung der Maximaltemperatur unabhängig von der Regulation der Heizelemente durch die Regelvorrichtung, also z.B. dem Mikroprozessor erfolgt. Sollte zur Regulation der Heizelemente beispielsweise auf der Regelvorrichtung eine Firmware installiert vorliegen, so ist es bevorzugt, dass der hardwareimplementierte Temperaturwächter auch bei einem Ausfall oder einem fehlerhaften Ausführen der Firmware die Maximaltemperatur der Behandlungsfläche zuverlässig begrenzt. Hierdurch kann durch einfache konstruktive Mittel besonders effektiv sichergestellt werden, dass die Behandlungsfläche der Vorrichtung eine Maximaltemperatur nicht überschreitet. Selbst unter Auftreten von Fehlsteuerungen in der Regelvorrichtung, beispielsweise nach dem Eintreten von Flüssigkeiten, kann aufgrund des hardwarebasierten Temperaturwächters vorteilhafterweise jederzeit gewährleistet werden, dass die Behandlungsfläche eine Maximaltemperatur nicht überschreitet. Durch dieses zusätzliche technische Element zur Temperaturüberwachung ist es möglich einen ausgezeichneten Sicherheitsstandard zu wahren, ohne mit der Funktionsweise der Vorrichtung zur hyperthermischen Behandlung zu interferieren.

Als weiteres Sicherheitselement weist die erfindungsgemäße Vorrichtung eine Schmelzsicherung auf, welche bei einem Kurzschluss der Vorrichtung oder ungeregeltem Durchheizen der Vorrichtung die Stromversorgung zur Vorrichtung unterbricht. Im Sinne der Erfindung wird unter einer Schmelzsicherung bevorzugt eine Überstromschutzeinrichtung verstanden, bei welcher beispielsweise durch das Abschmelzen eines Schmelzleiters ein Stromkreis unterbrochen werden kann, sobald die Stromstärke einen Grenzwert über eine zu bestimmende Zeit überschreitet. Es ist bevorzugt, dass die Schmelzsicherung in der Vorrichtung zwischen der Einspeisung der Versorgungsspannung in die Vorrichtung und der Vorrichtung selbst vorliegt. Sollte es zu einem Störfall kommen, welcher dadurch gekennzeichnet ist, dass ein unkontrollierter hoher Strom von der Versorgungseinspeisung in die Vorrichtung fließt, schaltet die Schmelzsicherung vorteilhafterweise die komplette Spannungsversorgung der Vorrichtung ab. Eine Schmelzsicherung bietet zum einen einen ausreichend schnellen, zum anderen einen extrem zuverlässigen Schutz.

Es hat sich gezeigt, dass selbst unter fehlerfreier Konstruktion der Vorrichtung und dem Bereitstellen eines hardwareimplementierten Temperaturwächters es nicht ausgeschlossen werden kann, dass es aufgrund einer fehlerhaften Bedienung oder Schädigung der Vorrichtung, in äußerst seltenen Fällen zu einem Durchheizen der Heizelemente kommt.
Unter dem Durchheizen der Heizelemente wird im Sinne der Erfindung bevorzugt verstanden, dass die Temperatur der Heizelemente unkontrolliert, d.h. nicht durch eine temperaturbasierte Regelung mithilfe der Regelvorrichtung steigt. Falls in diesen Störfällen der hardwareimplementierte Temperaturwächter versagt, kann die Behandlungsfläche unkontrolliert auf Temperaturen weit über der gewünschten Behandlungstemperatur ansteigen, beispielsweise auf Temperaturen von weit über 65 °C.

Obwohl ein solches fehlerhaftes Durchheizen äußerst selten auftritt, kann dies zu schwerwiegenden Schädigungen beim Patienten führen. Dies liegt insbesondere daran, dass die hyperthermisch zu behandelnden Hautpartien zumeist besonders empfindlich sind und beispielsweise durch eine Rötung, Schwellung oder gar Wundbildung gekennzeichnet sind. Eine deutlich erhöhte Temperatur über 65° kann lokal an diesen Stellen in besonderen Maße zu starken Schmerzen und Hautverbrennungen führen.

Im Hinblick auf die besonderen Umstände der Nutzung der Vorrichtung und damit verbundenen Sicherheitsanforderungen ist die genannte Schmelzsicherung besonders vorteilhaft, um noch für den unwahrscheinlichsten Fall einer Störung eine Abschaltung des Heizens der Behandlungsfläche gewährleisten zu können. So wird mithilfe der Schmelzsicherung unabhängig von jeglicher Temperaturmessung, aufgrund z.B. fehlerhafter Temperatursensoren, ein übermäßiges Heizen der Behandlungsfläche unterbunden. Es wurde erkannt, dass die Stromversorgung der Vorrichtung eine zentrale Regulationsschnittstelle darstellt, welche höchsten Sicherheitsanforderungen genügt. Durch eine Integration der Schmelzsicherung in den Stromfluss zur Versorgung der Vorrichtung kann sichergestellt werden, dass ein maximaler Versorgungsstrom während einer bestimmten Zeit nicht überschritten wird. Da ein Durchheizen und ein unkontrolliertes Heizen der Heizelemente über die gewünschte Temperatur mit einem erhöhten Stromfluss zusammenhängt, kann hierdurch auf besonders zuverlässige Weise ein Überhitzen der Behandlungsfläche vermieden werden. Insbesondere kann durch die Stromkontrolle sehr schnell reagiert werden, bevor der Strom so lange wirkt, dass er eine seiner Stärke entsprechende Temperatur erzeugt. Ein rein an der Temperatur orientierter letztinstanzlicher Sicherheitsmechanismus könnte außerdem aufgrund von thermischen Trägheiten der beteiligten Bauteile nicht schnell genug sein.

Bei der erfindungsgemäßen Vorrichtung zeigt sich eine besonders vorteilhafte und synergistische Wirkung der kombinierten Verwendung eines hardwareimplementierten Temperaturwächters und einer Schmelzsicherung.

So ist es ein Nachteil der Schmelzsicherung, dass diese nach dem einmaligen Auslösen eine permanente Abkopplung der Versorgungsspannung von der Vorrichtung zur Folge hat. Eine erneute Inbetriebnahme der Vorrichtung nach dem Auslösen der Schmelzsicherung bedarf der Reparatur durch einen Techniker, beispielsweise den Austausch der Schmelzsicherung. Im Hinblick auf die Kosten ist die Vorrichtung in der Regel bei einem Auslösen der Schmelzsicherung unbrauchbar geworden.

Vorteilhafterweise ist der hardwareimplementierte Temperaturwächter jedoch so eingestellt, dass es nicht zu einem permanenten Abschalten der Stromversorgung der Vorrichtung kommen muss. Vielmehr ist der hardwareimplementierte Temperaturwächter derart konzipiert, dass wenn die Temperatur der Behandlungsfläche eine Maximaltemperatur überschreitet, während des Zeitraumes des Überschreitens die Stromversorgung der Heizelemente unterbrochen wird. Die Stromunterbrechung durch den hardwareimplementierten Temperaturwächter ist somit vorteilhafterweise reversibel, d.h. sobald die Temperatur der Behandlungsfläche wieder unter die Maximaltemperatur fällt, können die Heizelemente wieder heizen.

So kann auch nach einem einmaligen Auftreten einer Fehlsteuerung die bestimmungsgemäße Benutzung der Vorrichtung fortgeführt werden. Der Benutzer würde gegebenenfalls nichts von der Fehlsteuerung bemerken, da durch die gewählte Maximaltemperatur, die Effektivität und die Unabhängigkeit des hardwareimplementierten Temperaturwächters keine für den Benutzer als unangenehm empfundenen Temperaturen entstehen und die Vorrichtung bei der nächsten Benutzung im Falle einer einmaligen Fehlsteuerung wieder einwandfrei funktioniert könnte.

Die Kombination der Sicherheitsmerkmale eines hardwareimplementierten Temperaturwächters mit einer Schmelzsicherung erlauben eine überraschend zuverlässige Kontrolle der Temperatur mit möglichst wirtschaftlichen Mitteln aufgrund gestaffelter Sicherheitsschranken. Insbesondere bei einer Vorrichtung, die mit einem Mobilgerät verbunden wird, ist eine Kombination von Sicherheitsschranken sinnvoll. Mobilgeräte können technischen Problemen ausgesetzt sein, welche einer sicheren Steuerung entgegenwirken. Beispielsweise können Mobilgeräte von Computerviren befallen sein. Dann ist es gut, wenn die Vorrichtung einen eigenen Sicherheitsmechanismus aufweist, welcher bevorzugt zweistufig ist.

Ein weiterer synergistischer Effekt durch die Kombination der Sicherheitsmerkmale eines hardwareimplementierten Temperaturwächters mit einer Schmelzsicherung kann darin gesehen werden, dass beispielsweise ein zwar unwahrscheinliches, jedoch mögliches einmaliges Versagen der Regelvorrichtung durch den hardwareimplementierten Temperaturwächter reversibel abgefangen wird. Sollte jedoch ein äußerst unwahrscheinliches größeres Problem auftreten, welches den hardwareimplementierten Temperaturwächter umfasst, so tritt die Schmelzsicherung als letztinstanzliche Absicherung in Aktion. Da diese jedoch irreversibel ist, kann keine unter diesen Umständen gefährliche Weiterbenutzung durch den Benutzer stattfinden, sondern es wird ein Gang zum Techniker bzw. zum Fachhandel veranlasst.

Bevorzugt erfolgt bereits mithilfe der Regelvorrichtung eine Temperierung der Behandlungsfläche. Falls die Regelvorrichtung aufgrund z.B. einer fehlerhaften Elektronik versagt, erlaubt der hardwareimplementierte Temperaturwächter ein Abschalten der Heizelemente unabhängig von der Regelvorrichtung. Selbst bei einer solchen Störung der Regelvorrichtung würde eine Schmelzsicherung nicht ausgelöst. Nur in dem äußerst seltenen Fall, dass sowohl die Regelvorrichtung, als auch der hardwareimplementierte Temperaturwächter versagt, beispielsweise bei einer Beschädigung der entsprechenden Bauelemente, gewährleistet die Schmelzsicherung ein finales Kontrollelement. Kommt es zu einem erhöhten Strombedarf der Heizelemente aufgrund eines starken Erhitzens, schaltet die Schmelzsicherung die gesamte Stromversorgung der Vorrichtung ab. Durch diese Staffelung der Sicherheitsmechanismen kann eine einmalige Störung der Regelvorrichtung äußerst sicher abgefangen werden. Der hardwareimplementierte Temperaturwächter schreitet unbemerkt und schnell ein, ohne die Benutzbarkeit der Vorrichtung zu beeinflussen. Durch die nachgeschaltete Schmelzsicherung kann ein noch höheres Sicherheitslevel erreicht werden, so dass dem Benutzer eine ungemein effektive und sichere Behandlungsvorrichtung zur Verfügung gestellt werden kann.

Durch eine Hintereinanderschaltung der Sicherheitsschranken kann überraschend gewährleistet werden, dass die Behandlungsfläche nicht in einen Temperaturbereich gelangt, welcher den Patienten gefährden könnte.

In einer bevorzugten Ausführungsform beträgt die Maximaltemperatur zwischen 54 °C und 58 °C, bevorzugt ungefähr 56 °C. Es hat sich überraschenderweise gezeigt, dass durch eine Maximaltemperatur von einem Wert zwischen 54 °C und 58 °C bevorzugt von ungefähr 56 °C weder der Behandlungserfolg durch die hyperthermische Behandlung gefährdet wird, noch ein unangenehmes Empfinden auf der Haut erzeugt wird, so dass diese Maximaltemperatur eine ideale erste Sicherheitsstufe zum Schutz vor Überhitzen darstellt.

In einer bevorzugten Ausführungsform umfasst der hardwareimplementierte Temperaturwächter mindestens einen zweiten Temperatursensor zur Messung der Temperatur der Behandlungsfläche und einen Komparator, wobei der Komparator die Temperatur der Behandlungsfläche mit der Maximaltemperatur vergleicht und bei Überschreiten der Maximaltemperatur die Stromzufuhr zu dem Heizelement unterbindet.

Im Sinne der Erfindung bezeichnet ein Komparator bevorzugt eine elektronische Schaltung zum Vergleichen zweier Spannungen, wobei am Ausgang in binärer Weise angezeigt wird, welcher der beiden Spannungen höher ist. Im Stand der Technik sind hinreichend verschiedene Komparatoren bekannt, welche geeignet sind aus zwei analogen Spannungen ein binäres Ausgangsignal auszugeben, welche aussagt, welche der Eingangsspannung höher ist. Als Beispiel für eine Komparator-Schaltung sei der Schmitt-Trigger genannt. Es ist bevorzugt, dass an einem Eingang des Komparators mit Hilfe eines Spannungsverteilers ein Referenzwert für eine Spannung angelegt wird. Dieser Referenzwert entspricht bevorzugt dem Spannungswert, welcher der zweite Temperatursensor aufweisen würde, wenn die Temperatur der Behandlungsfläche gleich der Maximaltemperatur ist. An dem zweiten Eingang des Komparators liegt bevorzugt die Ausgangsspannung des Temperatursensors an, welche von der Temperatur der Behandlungsfläche abhängt. Ein besonders bevorzugter Temperatursensor umfasst zu diesem Zweck einen NTC-Thermistor, d.h. einen Heißleiter. Dieser besitzt einen negativen Temperaturkoeffizienten, sodass bei einer Erhöhung der Temperatur der Widerstand fällt und ein höherer Strom fließt. Es können aber auch Kaltleiter, d.h. PTC-Thermistoren verwandt werden, welche einen positiven Temperaturkoeffizienten besitzen, sodass bei einer Erhöhung der Temperatur der Widerstand steigt und ein niedrigerer Strom fließt.

Steigt die Temperatur der Behandlungsfläche, bewegt sich der durch den zweiten Temperatursensor gesteuerte Spannungswert am Komparator auf den Referenzwert der Spannung zu, welcher der Maximaltemperatur entspricht. Sobald die Temperatur die Maximaltemperatur überschreitet, ändert sich das Ausgangsignal am Komparator binär. Der Komparator ist bevorzugt in die Stromversorgung der Heizelemente integriert. D.h. bevor die Temperatur der Behandlungsfläche die Maximaltemperatur erreicht, gibt der Komparator bevorzugt die Versorgungsspannung der Heizelemente frei. Sobald jedoch die Temperatur höher als die Maximaltemperatur ist, schaltet der Ausgang des Komparators ab und unterbricht die Versorgungsspannung der Heizelemente. Fällt die Temperatur der Behandlungsfläche wieder, wird die Versorgungsspannung vorteilhafterweise wieder durch den Komparator freigegeben. Hierdurch kann ein reversibles An- und Abschalten der Heizelemente nur für den Zeitraum erfolgen, während die Temperatur der Behandlungsfläche die Maximaltemperatur überschreitet. Weiterhin kann es bevorzugt sein, dass der Komparator durch die Regelvorrichtung beim Starten der Vorrichtung freigeschaltet wird. Erfolgt somit kein ordnungsgemäßer Start der Vorrichtung ist der Komparator in der Voreinstellung so konfiguriert, dass die Spannungsversorgung der Heizelemente unterbrochen ist.

Die beschriebene bevorzugte Ausführungsform des hardwareimplementierten Temperaturwächters hat sich in Tests als besonders robust und zuverlässig erwiesen. Aufgrund der Reversibilität der Sicherheitsabschaltung und einfachen Konstruktionsweise zeichnet sich die bevorzugte Ausführungsform weiterhin durch niedrige Herstellungs- und Wartungskosten aus.

Durch die von der Regelvorrichtung unabhängigen Bauweise samt eigenem Temperatursensor kann ein zuverlässiger Betrieb auch bei Ausfall eines Bauteils der Regelvorrichtung gewährleistet werden. Ebenso ist ein hardwareimplementierter Temperaturwächter in beschriebener Form unter Ausnutzung eines Komparators besonders schnell, da Komparatoren weit verbreitete elektronische Bauteile sind, die sich neben ihrer Zuverlässigkeit durch ihre schnelle Schaltfähigkeit ausweisen. So gibt es beispielsweise Komparatoren mit Schaltzeiten von Nanosekunden (ns) und darunter. Es konnte überraschenderweise festgestellt werden, dass durch den Einsatz von Komparatoren in der Schaltung aufgrund Ihrer Schnelligkeit ein besonders wirksamer Schutzmechanismus gegen Überhitzen der Behandlungsfläche aufgebaut werden konnte.

In einer bevorzugten Ausführungsform weist die Schmelzsicherung einen Schwellenwert für einen maximalen Strom auf, welcher der Erhitzung der Behandlungsfläche auf 65 °C für 1 Sekunde entspricht. Tests haben gezeigt, dass erst eine Temperaturerhöhung von über 65 °C für länger als 1 Sekunde sehr kritisch für das Schmerzempfinden ist und zu Schädigungen der Hautpartien führen kann. Vorteilhafterweise wird durch Einstellung der Schmelzsicherung auf diese Parameterwerte die Schmelzsicherung nicht vorzeitig bei unkritischen Temperaturerhöhungen der Behandlungsfläche ausgelöst. Hierdurch kann Wirtschaftlichkeit erhöht werden, ohne einen Kompromiss in Bezug auf die Sicherheit einzugehen. Der Fachmann weiß anhand der elektrischen Parameter der Heizelemente, welche Schmelzsicherung gewählt werden sollte, um die angebenden Werte zu gewährleisten. Hierzu kann auch eine Messung der Stromzufuhr bei gleichzeitiger Messung der Temperatur der Behandlungsfläche erfolgen. Weiterhin ist es besonders bevorzugt eine flinke Schmelzsicherung zu verwenden, welche auf eine Stromerhöhung innerhalb von bevorzugt weniger als 20 Millisekunden (ms) reagiert. So wurde erkannt, dass auch eine kurzfristige Stromerhöhung von weniger als 20 ms aufgrund der thermischen Trägheit der Behandlungsfläche zu einer Temperaturerhöhung von länger als 1 Sekunde führen kann.

Gegenüber nicht rückstellenden, rein temperaturabhängigen Thermosicherungen, welche ebenfalls durch Abschmelzen funktionieren, hat die hier verwendete stromabhängige Schmelzsicherung einige Vorteile. Bei nicht rückstellenden, reinen temperaturabhängigen Thermosicherungen geschieht das Abschmelzen nicht bei Anliegen eines Stromes oberhalb eines Schwellenwertes, sondern erst bei Anliegen einer externen Temperatur, welche größer ist als eine definierte Maximaltemperatur. Gegenüber nicht rückstellenden reinen temperaturabhängigen Thermosicherungen können stromabhängige Schmelzsicherungen somit schon reagieren, bevor in Folge eines länger wirkenden erhöhten Stroms eine bestimmte unerwünschte Temperatur überhaupt erreicht wird. Ebenso brauchen nicht rückstellende reine temperaturabhängige Thermosicherungen bei Anliegen einer externen Temperatur, welche größer ist als eine definierte Maximaltemperatur, immer eine gewisse Reaktionszeit. Somit kann es zu gefährlichen, weiteren Temperaturerhöhungen kommen. Stromabhängige Schmelzsicherungen reagieren im Gegensatz dazu schneller und mit minimalen systembedingten Latenzzeiten.

In einer bevorzugten Ausführungsform beträgt der Schwellenwert der Schmelzsicherung bevorzugt zwischen 1 A und 2,5 A besonders bevorzugt ungefähr 2 A. Tests haben gezeigt, dass in Bezug auf die bevorzugten Heizelemente die genannten Schwellenwerte besonders zuverlässig gewährleisten, dass die Temperatur der Behandlungsfläche eine Temperatur von 65 °C bis 70 °C für nicht länger als 1 Sekunde überschreitet. Durch das Schmelzen der Schmelzsicherung ab Stromwerten von 1 A bis 2.5 A kann somit sichergestellt werden, dass die Temperatur der Behandlungsfläche nicht in einen gesundheitsgefährdenden Bereich kommen kann. So kommt es bei einer regulären Behandlung zu einem regulären Behandlungsstrom, welcher unterhalb von 2,5 A, bevorzugt 1 A liegt. Tritt ein Fehler auf, z.B. bei einem Durchheizen, so fließt ein erhöhter Strom. In diese Falle Sicherung greift die Sicherung ein und verhindert wirksam ein unkontrolliertes Aufheizen.

Durch die vorteilhafte Auswahl der Maximaltemperatur des hardwareimplementierten Temperaturwächters auf einen Wert zwischen 54 °C und 58 °C bevorzugt von ungefähr 56 °C kann außerdem sichergestellt werden, dass der Abstand groß genug ist zur Temperatur beim Auslösen der Schmelzsicherung in Folge eines Stromwertes oberhalb des Schwellenwertes. So kann ein versehentliches Auslösen der Schmelzsicherung, welche mindestens einen Austausch der Sicherung zur Folge hätte, vermieden werden, so lange keine schwerwiegende Störung, welche den hardwareimplementierten Temperaturwächter einschließt, vorliegt.

In einem weiteren Aspekt betrifft die Erfindung ein System aus Vorrichtung und Mobilgerät, wobei die Vorrichtung konfiguriert ist für eine Stromversorgung durch das Mobilgerät und/oder zum Datenaustausch mit dem Mobilgerät und/oder zur Steuerung durch das Mobilgerät über ein Interface.

Dabei kann bevorzugt sein, dass eine Regelvorrichtung in der Vorrichtung umfasst ist, es kann aber auch bevorzugt sein, dass das Mobilgerät die Regelvorrichtung umfasst, insbesondere bei Steuerung durch das Mobilgerät über ein Interface.

Der durchschnittliche Fachmann erkennt, dass technische Merkmale und Vorteile bevorzugter Ausführungsformen der erfindungsgemäßen Vorrichtung auch für das erfindungsgemäße System gelten.

## Patentansprüche

1. Vorrichtung zur hyperthermischen Behandlung von Juckreiz umfassend mindestens eine Behandlungsfläche, welche durch Erhitzen mindestens eines Heizelements auf eine Behandlungstemperatur von 40 °C bis 65 °C aufheizbar ist und durch eine Regelvorrichtung steuerbar ist,
**dadurch gekennzeichnet, dass**
die Vorrichtung mindestens ein Interface aufweist und konfiguriert ist für eine Stromversorgung der Vorrichtung durch ein Mobilgerät und/oder zum Datenaustausch mit einem Mobilgerät und/oder zur Steuerung durch ein Mobilgerät über das Interface.

2. Vorrichtung nach Anspruch 1
**dadurch gekennzeichnet, dass**
die Vorrichtung die Regelvorrichtung umfasst.

3. Vorrichtung nach Anspruch 2,
**dadurch gekennzeichnet, dass**
die Vorrichtung konfiguriert ist für eine Parametrierung der Regelvorrichtung durch das Mobilgerät.

4. Vorrichtung nach Anspruch 2 oder 3,
**dadurch gekennzeichnet, dass**
die Vorrichtung konfiguriert ist für eine Übertragung eines Regelprogramms der Regelvorrichtung durch das Mobilgerät.

5. Vorrichtung nach Anspruch 1,
**dadurch gekennzeichnet, dass**
das Mobilgerät die Regelvorrichtung umfasst.

6. Vorrichtung nach einem oder mehreren der vorigen Ansprüche,
**dadurch gekennzeichnet, dass**
die Stromversorgung der Vorrichtung durch das Mobilgerät und/oder der Datenaustausch mit dem Mobilgerät und/oder die Steuerung durch das Mobilgerät über das Interface drahtlos erfolgt.

7. Vorrichtung nach einem oder mehreren der vorigen Ansprüche,
**dadurch gekennzeichnet, dass**
die Behandlungstemperatur 40 °C bis 60 °C beträgt und die Größe der Behandlungsfläche zwischen 1 cm² und 18 cm² beträgt.

8. Vorrichtung nach einem oder mehreren der vorigen Ansprüche,
**dadurch gekennzeichnet, dass**
die Behandlungstemperatur 40 °C bis 60 °C beträgt und die Größe der Behandlungsfläche kleiner als 1 cm² ist.

9. Vorrichtung nach einem oder mehreren der vorigen Ansprüche,
**dadurch gekennzeichnet, dass**
die Vorrichtung einen Energiespeicher, bevorzugt ein Akku, besonders bevorzugt ein Lithium-Polymer und/oder ein Metall-hydrid-Akku umfasst.

10. Vorrichtung nach Anspruch 9, wobei der Akku ein Festkörperakku, bevorzugt ein Lithium - Keramik - Akku ist.

11. Vorrichtung gemäß einem oder mehreren der vorigen Ansprüche
**dadurch gekennzeichnet, dass**
die Vorrichtung mindestens einen ersten Temperatursensor zur Messung der Temperatur der Behandlungsfläche umfasst und die Regelvorrichtung und/oder Steuerung basierend auf den Messdaten des Temperatursensors die Temperatur des mindestens einen Heizelementes einstellt.

12. Vorrichtung nach einem oder mehreren der vorigen Ansprüche, wobei eine Behandlungstemperatur für eine Behandlungsdauer von 1 - 10 sec aufrechterhalten wird.

13. Vorrichtung nach einem oder mehreren der vorigen Ansprüche, wobei ein hardwareimplementierter Temperaturwächter die Maximaltemperatur der Behandlungsfläche reversibel begrenzt und eine Schmelzsicherung bei Kurzschluss oder ungeregeltem Durchheizen die Vorrichtung abschaltet.

14. Vorrichtung nach Anspruch 13, wobei der hardwareimplementierte Temperaturwächter mindestens einen zweiten Temperatursensor zur Messung der Temperatur der Behandlungsfläche und einen Komparator umfasst, wobei der Komparator die Temperatur der Behandlungsfläche mit der Maximaltemperatur vergleicht und bei Überschreiten der Maximaltemperatur die Stromzufuhr zu dem Heizelement unterbindet.

15. System aus Vorrichtung und Mobilgerät, wobei die Vorrichtung konfiguriert ist für eine Stromversorgung durch das Mobilgerät und/oder zum Datenaustausch mit dem Mobilgerät und/oder zur Steuerung durch das Mobilgerät über ein Interface.
